# EUROPEAN PATENT SPECIFICATION

(11) **EP 2 892 911 B1**
(45) Date of publication and mention of the grant of the patent: **30.08.2017**
(21) Application number: 13767175.6
(22) Date of filing: 10.09.2013
(51) Int. Cl.: C07J 63/00, A61K 31/56, A61P 29/00

(54) **C17-HETEROARYL DERIVATIVES OF OLEANOLIC ACID AND METHODS OF USE THEREOF**
C17-HETEROARYL-DERIVATE VON OLEANOLSÄURE UND VERFAHREN ZUR VERWENDUNG DAVON
DÉRIVÉS C17-HÉTÉROARYLÉS DE L'ACIDE OLÉANOLIQUE ET LEURS PROCÉDÉS D'UTILISATION

(30) Priority: 10.09.2012 US 201261699199 P
(43) Date of publication of application: 15.07.2015
(73) Proprietor: Reata Pharmaceuticals, Inc., Irving, TX 75063-2648 (US)
(72) Inventor: JIANG, Xin, Irving, TX 75063-2648 (US); BENDER, Christopher, F., Irving, TX 75063-2648 (US); VISNICK, Melean, Irving, TX 75063-2648 (US)
(74) Representative: Vossius & Partner Patentanwälte Rechtsanwälte mbB
(86) International application number: PCT/US2013/059015
(87) International publication number: WO 2014/040056

(56) References cited:
- WO-A1-2009/129545
- WO-A1-2009/129548
- WO-A1-2012/125488

### SEQUENCE LISTING

<110> REATA PHARMACEUTICALS, INC.
<120> C17-HETEROARYL DERIVATIVES OF OLEANOLIC ACID AND METHODS OF USE THEREOF
<130> REAT.P0076WO
<140> UNKNOWN
   <141> 2013-09-10
<150> 61/699,199
   <151> 2012-09-10
<160> 1
<170> PatentIn version 3.5
<210> 1
   <211> 26
   <212> DNA
   <213> Artificial Sequence
<220>
   <223> Synthetic primer
<400> 1
   cagtcacagt gactcagcag aatctg 26

## Description

### BACKGROUND OF THE INVENTION

### I. Field of the Invention

The present invention relates generally to the fields of biology and medicine. More particularly, it concerns compounds, compositions and methods for the treatment and prevention of diseases such as those associated with oxidative stress and inflammation.

### It Description of Related Art

The anti-inflammatory and anti-proliferative activity of the naturally occurring triterpenoid, oleanolic acid, has been improved by chemical modifications. For example, 2-cyano-3,12-diooxooleana-1,9(11)-dien-28oie acid (CDDO) and related compounds have been developed (Honda *et al*., 1997; Honda *et al.,* 1998; Honda *et al.*, 1999; Honda *et al*., 2000a; Honda *et al*., 2000b; Honda, *et al.*, 2002; Suh *et al.* 1998; Suh *et al*., 1999; Place *et al.* 2003; Liby *et al.,* 2005; and U.S. Patents 8,129,429; 7,915,402; 8,124,799; 8,071,632; 8,338,618; and 7,943,778), The methyl ester, bardoxolone methyl (CDDO-Me), has been evaluated clinically for the treatment of cancer and chronic kidney disease (Pergola *et al*., 2011; Hong *et al*., 2012).

Synthetic triterpenoid analogs of oleanolic acid have also been shown to be inhibitors of cellular inflammatory processes, such as the induction by IFN-γ of inducible nitric oxide synthase (iNOS) and of COX-2 in mouse macrophages. See Honda *et al*. (2000a); Honda *et al*. (2000b), and Honda *et al*. (2002). Synthetic derivatives of another triterpenoid, betulinie acid, have also been shown to inhibit cellular inflammatory processes, although these compounds have been less extensively characterized (Honda *et al.*, 2006). The pharmacology of these synthetic trterpenoid molecules is complex, Compounds derived from oleanolie acid have been shown to affect the function of multiple protein targets and thereby modulate the activity of several important cellular signaling pathways related to oxidative stress, cell cycle control, and inflammation (*e.g*., Dinkova-Kostova *et al*., 2005: Ahmad *et al*., 2006; Ahmad *et al*., 2008; Liby *et al*., 2007a). Derivatives of betulinie acid, though they have shown comparable anti-inflammatory properties, also appear to have significant deferences in their pharmacology compared to OA-derived compounds (Liby *et al*., 2007b). Given that the biological activity profiles of known triterpenoid derivatives vary, and in view of the wide variety of diseases that may bye treated or prevented with compounds having potent antioxidant and anti-inflammatory effects, and the high degree of unmet medical need represented within this variety of diseases, it is desirable to synthesize new compounds with diverse structures that may have improved biological activity profiles for the treatment of one or more indicaitons. WO-A-2009/129545 and WO-A-2009/129548 disclose oleanane compounds substituted in position 17 by heterocyclic groups, which are saturated in position 9(11) and having anti-inflammatory activity.

### SUMMARY OF THE INVENTION

The present disclosure provides novel synthetic triterpenoid derivatives with anti-inflammatory and/or antioxidant properties, pharmaceutical compositions, and methods for their manufacture, and methods for their use.

In one aspect, there are provided compounds of the formula: 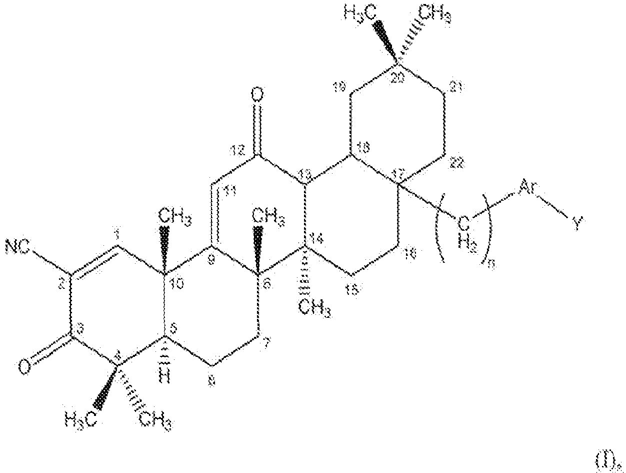 wherein:
n is 0-3:
Ar is heteroarenediyl_{(C≤8)} or a substituted version thereof; and
Y is; hydrogen, hydroxy, halo, amino, or cyano or -NCO; or alkyl_{(C≤8)} cycloalkyl, alkenyl_{(C≤8)}, alkynyl_{(C≤8)}, aryl_{(C≤12)}, aralkyl_{(C≤12)}, heteroaryl_{(C≤8)} heteroccloalkyl_{(C≤12)}, acyl_{(C≤12)}, alkoxy_{(C≤8)}, aryloxy_{(C≤12)}, acyloxy_{(C≤8)}, alkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, or substituted versions of any of these groups;
or a pharmaceutically acceptable salt thereof.

In some embodiments, Y is -H. In some embodiments, Y is alkyl_{(C≤4)}, for example, methyl *n*-propyl, isopropyl or cyclopropyl. In some embodiments, Y is substituted alkyl_{(C≤4)}, for example, methoxymethyl.

In some embodiments, Ar is

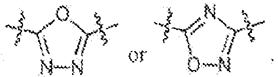

In some embodiments, n = 0. In other embodiments, n = 1.

In some embodiment, the compound is selected from the group consisting of: 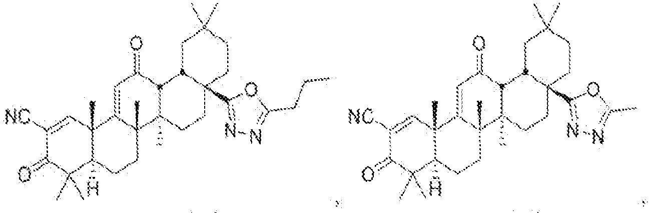 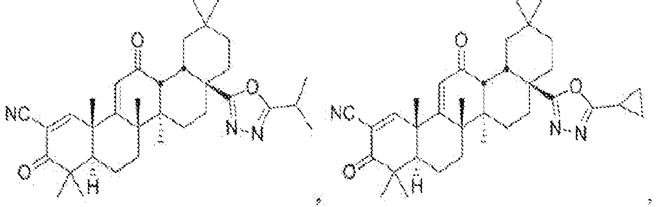 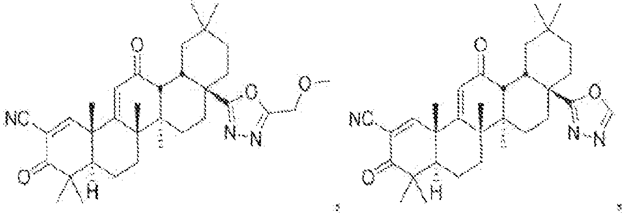 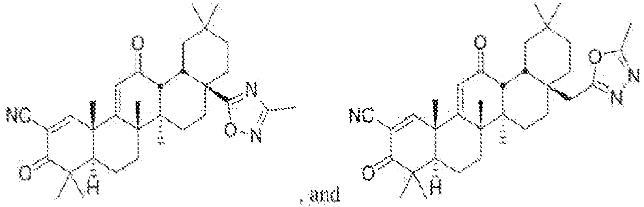 or a pharmaceutically acceptable salt of any of the above formulas.

In some aspects, there are provided pharmaceutical compositions comprising one or more of the above compounds and an excipient. In other aspect there are provided methods of treating and/or preventing a disease or a disorder in patients in need thereof, comprising administering to such patients one or more of the above compounds in an amount sufficient to treat And/or prevent the disease or disorder.

### DESCRIPTION OF ILLUSTRATIVE EMBODIMENTS

Disclosed herein are new compounds and compositions with antioxidant and/or anti-inflammatory properties, methods for their manufacture, and methods for their use, including for the treatment and/or prevention of disease.

### I. Definitions

When used in the context of a chemical group: "hydrogen" means -H: "hydroxy" means -OH; "oxo" means =O; "carbonyl" means -C(=O)-; "carboxy" means -C(=O)OH (also written as -COOH or -CO₂H); "halo" means independently -F, -Cl, -Br or -I; "amino" -NH₂; "hydroxyamino" means -NHOH; "nitro" means -NO₂; imino means =NH; "cyano" means -CN; "isocyanate" means "-N=C=O; "azido" means -N₃; in a monovalent context "phosphate" means -OP(O)(OH)₂ or a deprotonated form thereof; in a divalent context "phosphate" means -OP(O)(OH)O- or a deprotonated form thereof; "mercapto" means -SH; and "thio" means =S; "sulfonyl" means -S(O)₂-; and "sulfinyl" means -S(O)-,

In the context of chemical formulas, the symbol "-" means a single bond, "=" means a double bond and "≡" means triple bond. The symbol "----" represents an optional bond, which if present is either single or double. The symbol "-̅-̅-̅-̅"̅ represents a single bond or a double bond. Thus, for example, the formula 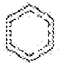 includes 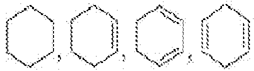 and 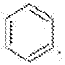 And it is understood that no one such ring atom forms part of more than one double bond. Furthermore, it is noted that the covalent bond symbol "-", when connecting one or two stereogenic atoms, does not indicate any preferred stereochemistry, instead, it cover all stereoisomers as well as mixtures thereof. The symbol " 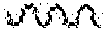 ", when drawn perpendicularly across a bond (*e.g*., 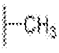 for methyl) indicates a point of attachment of the group. It is noted that the point of attachment is typically only identified in this manner for larger groups in order to assist the reader in unambiguously identifying point of attachment. The symbol " 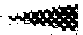 " means a single bond where the group attached to the thick end of the wedge is "out of the page." The symbol "means a single bond where the group attached to the thick end of the wedge is "into the page". The symbol " 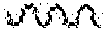 " means a single bond where the geometry around a double bond (*e.g*., either *E* or *Z*) is undefined. Both options, as well as combinations thereof are therefore intended. The bond orders described above are not limiting when one of the atoms connected by the bond is a metal atom (M).. In such cases, it is understood that the actual bonding may comprise significant multiple bonding and/or tonic character. Therefore, unless indicated otherwise, the formulas M-C, M=C, M----C, and M -̅-̅-̅-̅C, each refers to a bond of any and type and order between a metal atom and a carbon atom. Any undefined valency on an atom of a structure shown in this application implicitly represents a hydrogen atom bonded to that atom. A bold dot on a carbon atom indicates that the hydrogen attached to that carbon is oriented out of the plane of the paper.

When a group "R" is depicted as a "Boating group" on a ring system, for example in the formula; 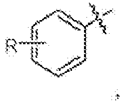 then R may replace any hydrogen atom attached to any of the ring atoms, including a depicted, implied, or expressly defined hydrogen, so long as a stable structure is formed. When a group "R" is depicted as a "floating group" on a fused ring system, as for example in the formula: 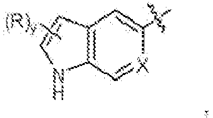 then R may replace any hydrogen attached to any of the ring atoms of either of the fused rings unless specified otherwise. Replaceable hydrogens include depicted hydrogens (*e.g*., the hydrogen attached to the nitrogen in the formula above), implied hydrogens (*e.g*., a hydrogen of the formula above that is not shown but understood to be present), expressly defined hydrogens, and optional hydrogens whose presence depends on the identity of a ring atom (*e.g*., a hydrogen attached to group X, when X equals -CH-), so long as a stable structure is formed. In the example depicted, R may reside on either the 5-membered or the 6-membered ring of the fused ring system, in the formula above, the subscript letter "y" immediately following the group "R" enclosed in parentheses, represents a numeric variable. Unless specified otherwise, this variable can be 0, 1, 2, or any integer greater than 2, only limited by the maximum number of replaceable hydrogen atoms of the ring or ring system.

For the groups and classes below, the following parenthetical subscripts further define the group/class as follows: "(Cn)" defines the exact number (n) of carbon atoms in the group/class. "(C≤n)" defines the maximum number (n) of carbon atoms that can be in the group/class, with the minimum number as small as possible for the group in question, *e.g.,* it is understood that the minimum number of carbon atoms in the group "alkenyl_{(C≤8)}" or the class "alkene_{(C≤8)}" is two. For example, "alkoxy_{(C≤10)}" designates those alkoxy groups having from 1 to 10 carbon atoms. (Cn-n') defines both the minimum (n) and maximum number (n') of carbon atoms in the group. Similarly, "alkyl_{(C2-10)}" designates those alkyl groups having from 2 to 10 carbon atoms.

The term "saturated" as used herein means the compound or group so modified has no carbon-carbon double and no carbon-carbon triple bonds, except as noted below. In the case of substituted versions of saturated groups, one or more carbon oxygen double bond or a carbon nitrogen double bond may be present. And when such a bond is present, then carbon-carbon double bonds that may occur as part of keto-enol tautomerism or imine/enamine tautomerism are not precluded.

The term "aliphatic" when used without the "substituted" modifier signifies that the compound/group so modified is an acyclic or cyclic, but non-aromatic hydrocarbon compound or group. In aliphatic compounds/groups, the carbon atoms can be joined together in straight chains, branched chains, or non-aromatic rings (alicyclic). Aliphatic compounds/groups can be saturated, that is joined by single bonds (alkanes/alkyl), or unsaturated, with one or more double bonds (alkenes/alkenyl) or with one or more triple bonds (alkynes/alkynyl).

The term "alkyl" when used without the "substituted" modifier refers to a monovalent saturated aliphatic group with a carbon atom as the point of attachment, a linear or branched acyclic structure, and no atoms other than carbon and hydrogen. Similarly, the cycloalkyl group consists of no atoms other than carbon and hydrogen. As used herein, the term does not preclude the presence of one or more alkyl groups (carbon number limitation permitting) attached to the ring or ring system. The groups -CH₃ (Me), -CH₂CH₃ (Et), -CH₂CH₂CH₃ (*n*-Pr or propyl), -CH(CH₃)₂ (i-Pr, *ⁱ*Pr or isopropyl), -CH(CH₂)₂ (cyclopropyl), -CH₂CH₂CH₂CH₃ (*n*-Bu), -CH(CH₃)CH₂CH₃ (*sec*-butyl), -CH₂CH(CH₃)₂ (isobutyl), -C(CH₃)₃ (*tert*-butyl, *t*-butyl, *t*-Bu or *^{t}*Bu), -CH₂C(CH₃)₃ (*neo*-pentyl), cyclobutyl, cyclopentyl, cyclohexyl, and cyclohexylmethyl are non-limiting examples of alkyl and cycloalkyl groups. The term "alkanediyl" when used without the "substituted" modifier refers to a divalent saturated aliphatic group, with one or two saturated carbon atom(s) as the point(s) of
attachment, a linear or branched acyclic structure, no carbon-carbon double or triple bonds, and no atoms other than carbon and hydrogen. The groups, -CH₂- (methylene), -CH₂CH₂-, -CH₂C(CH₃)₂CH₂-, and -CH₂CH₂CH₂- are non-limiting examples of alkanediyl groups. The term "alkylidene" when used without the "substituted" modifier refers to the divalent group =CRR' in which R and R' are independently hydrogen, alkyl, or R and R' are taken together to represent an alkanediyl having at least two carbon atoms. Non-limiting examples of alkylidene groups include: =CH₂, =CH(CH₂CH₃), and =C(CH₃)₂. An "alkane" refers to the compound H-R, wherein R is alkyl as this term is defined above. When any of these terms is used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂. The following groups are non-limiting examples of substituted alkyl groups: -CH₂OH, -CH₂Cl, -CF₃, -CH₂CN, -CH₂C(O)OH, -CH₂C(O)OCH₃, -CH₂C(O)NH₂, -CH₂C(O)CH₃, -CH₂OCH₃, -CH₂OC(O)CH₃, -CH₂NH₂, -CH₂N(CH₃)₂, and -CH₂CH₂Cl. The term "haloalkyl" is a subset of substituted alkyl, in which one or more hydrogen atoms has been substituted with a halo group and no other atoms aside from carbon, hydrogen and halogen are present. The group, -CH₂Cl is a non-limiting example of a haloalkyl. The term "fluoroalkyl" is a subset of substituted alkyl, in which one or more hydrogen has been substituted with a fluoro group and no other atoms aside from carbon, hydrogen and fluorine are present. The groups, -CH₂F, -CF₃, and -CH₂CF₃ are non-limiting examples of fluoroalkyl groups.

The term "alkenyl" when used without the "substituted" modifier refers to an monovalent unsaturated aliphatic group with a carbon atom as the point of attachment, a linear or branched acyclic structure, at least one nonaromatic carbon-carbon double bond, no carbon-carbon triple bonds, and no atoms other than carbon and hydrogen. Non-limiting examples of alkenyl groups include: -CH=CH₂ (vinyl), -CH=CHCH₃, -CH=CHCH₂CH₃, -CH₂CH=CH₂ (allyl), -CH₂CH=CHCH₃, and -CH=CHCH=CH₂. The term "alkenediyl" when used without the "substituted" modifier refers to a divalent unsaturated aliphatic group, with two carbon atoms as points of attachment, a linear or branched acyclic structure, at least one nonaromatic carbon-carbon double bond, no carbon-carbon triple bonds, and no atoms other than carbon and hydrogen. The groups, -CH=CH-, -CH=C(CH₃)CH₂-, and -CH=CHCH₂- are non-limiting
examples of alkenediyl groups. It is noted that while the alkenediyl group is aliphatic, once connected at both ends, this group is not precluded from forming part of an aromatic structure. The terms "alkene" or "olefin" are synonymous and refer to a compound having the formula H-R, wherein R is alkenyl as this term is defined above. A "terminal alkene" refers to an alkene having just one carbon-carbon double bond, wherein that bond forms a vinyl group at one end of the molecule. When any of these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂. The groups, -CH=CHF, -CH=CHCl and -CH=CHBr, are non-limiting examples of substituted alkenyl groups.

The term "alkynyl" when used without the "substituted" modifier refers to an monovalent unsaturated aliphatic group with a carbon atom as the point of attachment, a linear or branched acyclic structure, at least one carbon-carbon triple bond, and no atoms other than carbon and hydrogen. As used herein, the term alkynyl does not preclude the presence of one or more non-aromatic carbon-carbon double bonds. The groups, -C≡CH, -C≡CCH₃, and -CH₂C≡CCH₃, are non-limiting examples of alkynyl groups. An "alkyne" refers to the compound H-R, wherein R is alkynyl. When any of these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂.

The term "aryl" when used without the "substituted" modifier refers to a monovalent unsaturated aromatic group with an aromatic carbon atom as the point of attachment, said carbon atom forming part of a one or more six-membered aromatic ring structure, wherein the ring atoms are all carbon, and wherein the group consists of no atoms other than carbon and hydrogen. If more than one ring is present, the rings may be fused or unfused. As used herein, the term does not preclude the presence of one or more alkyl or aralkyl groups (carbon number limitation permitting) attached to the first aromatic ring or any additional aromatic ring present. Non-limiting examples of aryl groups include phenyl (Ph), methylphenyl, (dimethyl)phenyl, -C₆H₄CH₂CH₃ (ethylphenyl), naphthyl, and a monovalent group derived from biphenyl. The term "arenediyl" when used without the "substituted" modifier refers to a divalent aromatic group with two aromatic carbon atoms as points of attachment, said carbon atoms forming part of one or more six-membered aromatic ring structure(s) wherein the ring atoms are all carbon, and wherein the monovalent, group consists of no atoms other than carbon and hydrogen. As used herein, the term does not preclude the presence of one or more alkyl, aryl or aralkyl groups (carbon number limitation permitting) attached to the first aromatic ring or any additional aromatic ring present. If more than one ring is present, the rings may be fused or unfused. Unfused rings may be connected via one or more of the following a covalent bond, alkanediyl, or alkenediyl groups (carbon number limitation permitting). Non-limiting examples of arenediyl groups include: 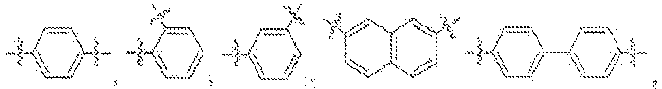 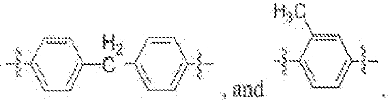 An "arene" refers to the compound H-R, wherein R is aryl as that term is defined above. Benzene and toluene are non-limiting examples of arenes. When any of these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₃,

The term "aralkyl" when used without the "substituted" modifier refers to the monovalent group -alkanediyl-aryl, in which the terms alkanediyl and aryl are each used in a manner consistent with the definitions provided above. Non-limiting examples of aralkyls are; phenylmethyl (benzyl, Bn) and 2-phenyl-ethyl. When the term aralkyl is used with the "substituted" modifier one or more hydrogen atom from the alkanediyl and/or the aryl group has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂. Non-limiting examples of substituted aralkyls are: (3'-chlorohenyl)-methyl, and 2-chloro-2-phenyl-eth-1-yl.

The term "heteroaryl" when used without the "substituted" modifier refers to a monovalent aromatic group with an aromatic carbon atom or nitrogen atom as the point of attachment, said carbon atom or nitrogen atom forming part of one or more aromatic ring structures wherein at least one of the ring atoms is nitrogen, oxygen or sulfure, and wherein the heteroaryl group consists of no atoms other than carbon, hydrogen, aromatic nitrogen, aromatic oxygen and aromatic sulfur. If more than one ring is present, the rings may be fused or unfused. As used herein, the term does not preclude the presence of one or more alkyl, aryl, and/or aralkyl groups (carbon number limitation permitting) attached to the aromatic ring or aromatic ring system. Non-limiting examples of heteroaryl groups include furanyl, imidazolyl, indolyl, indazolyl (Im), isoxazolyl, methylpyridinyl, oxazolyl, phenylpyridinyl, pyridinyl, pyrrolyl, pyrimidinyl, pyrazinyl, quinolyl, quinazolyl, quinoxalinyl, triazinyl, tetrazolyl, thiazolyl, thienyl, and triazolyl. The term "*N*-heteroaryl" refers to a heteroaryl group with a nitrogen atom as the point of attachment. The term "heteroarenediyl" when used without the "substituted" modifier refers to an divalent aromatic group, with two aromatic carbon atoms, two aromatic nitrogen atoms, or one aromatic carbon atom and one aromatic nitrogen atom as the two points of attachment, said atoms forming par of one or more aromatic ring structure(s) wherein at least one of the ring atoms is nitrogen, oxygen or sulfur, and wherein the divalent group consists of no atoms other than carbon, hydrogen, aromatic nitrogen, aromatic oxygen and aromatic sulfur. If more than one ring is present, the rings may be fused or unfused. Unfused rings may be connected via one or more of the following: a covalent bond, alkanediyl, or alkenediyl groups (carbon number limitation permitting). As used herein, the term does not preclude the presence of one or more alkyl, aryl, and/or aralkyl groups (carbon number limitation permitting) attached to the aromatic ring or aromatic ring system. Non-limiting examples of heteroarenediyl groups include: 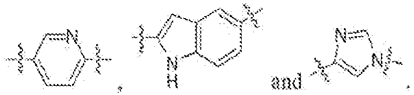 A "heteroarene" refers to the compound H-R, wherein R is heteroaryl. Pyridine and quinoline are non-limiting examples of heteroarenes. When these terms are used with the "substituted" modifier one or more hydrogen atom has been independently replaced by -OH, -F, -Cl, Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂ -C(O)NH₂, -OC(O)CH₃, or -S(O)₂NH₂,

The term "heterocycloalkyl" when used without the "substituted" modifier refers to a monovalent non-aromatic group with a carbon atom or nitrogen atom as the point of attachment, said carbon atom or nitrogen atom forming part of one or more non-aromatic ring structures wherein at least one of the ring atoms is nitrogen, oxygen or sulfur, and wherein the heterocycloalkyl group consists of no atoms other than carbon, hydrogen, nitrogen, oxygen, and sulfur. If more than one ring is present, the rings may be fused or unfused. As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkyl groups include aziridinyl, azetidinyl, pyrrolidinyl, piperidinyl, piperazinyl, morpholinyl, thiomorpholinyl, tetrahydrofuranyl, tetrahydrothiofuranyl, tetrahydropyranyl, pyranyl, oxiranyl, and oxetanyl. The term "N-heterocycloalkyl" refers to a heterocycloalkyl group with a nitrogen atom as the point of attachment. The term "heterocycloalkanediyl" when used without the "substituted" modifier refers to an divalent cyclic group, with two carbon atoms, two nitrogen atoms, or one carbon atom and one nitrogen atom as the two points of attachment, said atoms forming part of one or more ring structure(s) wherein at least one of the ring atoms is nitrogen, oxygen or sulfur, and wherein the divalent group consists of no atoms other than carbon, hydrogen, nitrogen, oxygen and sulfur. If more than one ring is present, the rings may be fused or unfused. Unfused rings may be connected via one or more of the following: a covalent bond, alkanediyl, or alkenediyl groups (carbon number limitation permitting). As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkanediyl groups include: 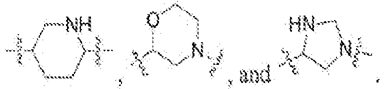 When these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, -S(O)₂NH₃ or -C(O)OC(CH₃)₃ (*tert*-butyloxycarbonyl, BOC).

The term "aryl" when used without the "substituted" modifier refers to the group -C(O)R, in in which R is a hydrogen, alkyl, aryl, aralkyl or heteroaryl, as those terms are defined above. The groups, -CHO, -C(O)CH₃ (acetyl, Ac), -C(O)CH₃CH₃. -C(O)CH₂CH₂CH₃, -C(O)CH(CH₃)₂, -C(O)CH(CH₂)₂, -C(O)C₆H₅, -C(O)C₆H₄CH₃, -C(O)CH₂C₆H₅, -C(O)(imidazolyl) are non-limiting examples of acyl groups. A "thioacyl" is defined in an analogous manner, except that the oxygen atom of the group -C(O)R has been replaced with a sulfur atom, -C(S)R. The term "aldehyde" corresponds to an alkane, as defined above, wherein at least one of the hydrogen atoms has been replaced with a -CHO group. When any of these terms are used with the "substituted" modifier one or more hydrogen atom (including a hydrogen atom directly attached the carbonyl or thiocarbonyl group, if any) has been independently replaced by -OH, -F, -Cl, -Br, -I, -NH₂, -NO₂, -CO₂H, -CO₂CH₃, -CN, -SH, -OCH₃, -OCH₃CH₃, -C(O)CH₃, -NHCH₃, -NHCH₂CH₃, -N(CH₃)₂, -C(O)NH₂, -OC(O)CH₃, or -S(O)₂NH₂. The groups, -C(O)CH₂CF₃, -CO₂H (carboxyl), -CO₂CH₃ (methycarboxyl), -CO₂CH₂CH₃, -C(O)NH₂ (carbamoyl), and -CON(CH₃)₂, are non-limiting examples of substituted acyl groups.

The term "alkoxy" when used without the "substituted" modifier refers to the group -OR, in which R is an alkyl, as that term is defined above. Non-limiting examples of alkoxy groups include: -OCH₃ (methoxy), -OCH₂CH₃ (ethoxy), -OCH₂CH₂CH₃, -OCH(CH₃)₂ (isopropoxy), -O(CH₃)₃ (*tert*-butoxy), -OCH(CH₂)₂, -O-cyclopentyl, and -O-cyclohexyl. The terms "alkenyloxy", "alkynyloxy", "aryloxy", "aralkoxy", "heteroaryloxy", "heterocycloalkoxy", and "acyloxy", when used without the "substituted" modifier, refers to groups, defined as -OR, in which R is alkenyl, alkynyl, aryl, aralkyl, heteroaryl, heterocycloalkyl, and acyl, respectively. The term "alkoxydiyl" refers to the divalent group -O-alkanediyl-, -O-alkanediyl-O-, or -alkanediyl-O-alkanediyl-. The term "alkylthio" and "acylthio" when used without the "substituted" modifier refers to the group -SR, in which R is an alkyl and acyl, respectively. The term "alcohol" corresponds to an alkane, as defined above, wherein at least one of the hydrogen atoms has been replaced with a hydroxy group. The term "ether" corresponds to an alkane, as defined above, wherein at least one of the hydrogen atoms has been replaced with an alkoxy group. When any of these terms is used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH_{3,} -HCH₂CH₃, -N(CH₃)₂, -C(O)NH₂, -OC(O)CH₃, or -S(O)₃NH₂.

The term "alkylamino" when used without the "substituted" modifier refers to the group -NHR, in which R is an alkyl, as that term is defined above. Non-limiting examples of alkylamino groups include: -NHCH₃ and -NHCH₂CH₃. The term "dialkylamino" when used without the "substituted" modifier refers to the group -NRR', in which R and R' can be the same or different alkyl groups, or R and R' can be taken together to represent an alkanediyl. Non-limiting examples of dialkylamino groups include: -N(CH₃)₂, -N(CH₃)(CH₂CH₃), and *N*-pyrrolidinyl. The terms "alkoxyamino", "alkenylamino", "alkynylamino", "arylamino", "aralkylamino", "heteroarylamino", "heterocycloalkylamino" and "alkylsulfonylamino" when used without the "substituted" modifier, refers to groups, defined as -NHR, in which R is alkoxy, alkenyl, alkynyl, aryl, aralkyl, heteroaryl, heterocycloalkyl, and alkylsulfonyl, respectively. A non-limiting example of an arylamino group is -NHC₆H₅. The term "amido" (acylamino), when used without the "substituted" modifier, refers to the group -NHR, in which R is acyl, as that term is defined above. A non-limiting example of an amido group is -NHC(O)CH₃. The term alkylimino" when used without the "substituted" modifier refers to the divalent group =NR, in which R is an alkyl, as that term is defined above. The term "alkylaminodiyl" refers to the divalent group -NH-alkanediyl-, -NH-alkanediyl-NH-, or -alkanediyl-NH-alkanediyl-. When any of these terms is used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂. The groups -NHC(O)OCH₃ and -NHC(O)NHCH₃ are non-limiting examples of substituted amido groups.

The terms "alkylsulfonyl" and "alkylsulfinyl" when used without the "substituted" modifier refers to the groups -S(O)₂R and -S(O)R, respectively, in which R is an alkyl, as that term, is defined above. The terms "alkenylsulfonyl", "alkynylsulfonyl", "arylsulfonyl", "aralkylsulfonyl", "heteroarylsulfonyl", and "heterocycloalkylsulfonyl" are defined in an analogous manner. When any of these terms is used with the "substituted" modifier one or more hydrogen atom has been independently replaced by -OH, -F, -Cl, -Br, -I, -NH₂, -NO_{2,} -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂.

The term "alkylphosphate" when used without the "substituted" modifier refers to the group -OP(O)(OH)(OR), in which R is an alkyl, as that term is defined above. Non-limiting examples of alkylphosphate groups include: -OP(O)(OH)(OMe) and -OP(O)(OH)(OEt), The term "dialkylphosphate" when used without the "substituted" modifier refers to the group -OP(O)(OR)(OR'), in which R and R' can be the same or different alkyl groups, or R and R' can be taken together to represent an alkanedyl. Non-limiting examples of dialkylphosphate groups include: -OP(O)(OMe)₂, -OP(O)(OEt)(OMe) and -OP(O)(OEt)₂. When any of these terms is used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂,

The use of the word "a" or "an," when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects.

As used herein, a "chiral auxiliary" refers to a removable chiral group that is capable of influencing the stereoselectivity of a reaction. Persons of skill in the art are familiar with such compounds, and many are commercially available.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

The term "effective," as that term is used in the specification and/or claims, means adequate to accomplish a desired, expected, or intended result. "Effective amount," "Therapeutically effective amount" or "pharmaceutically effective amount" when used in the context of treating a patient or subject with a compound means that amount of the compound which, when administered to a subject or patient for treating a disease, is sufficient to effect such treatment for the disease.

As used herein, the term "IC₅₀" refers to an inhibitory dose which is 50% of the maximum response obtained. This quantitative measure indicates how much of a particular drug or other substance (inhibitor) is needed to inhibit a given biological, biochemical or chemical process (or component of a process, i.e. an enzyme, cell, cell receptor or microorganism) by half.

An "isomer" of a first compound is a separate compound in which each molecule contains the same constituent atoms as the first compound, but where the configuration of those atoms in three dimensions differs.

As used herein, the term "patient" or "subject" refers to a living mamalian organism, such as a human, monkey, cow, sheep, goat, dog, cat, mouse, rat, guinea pig, or transgenic species thereof. In certain embodiments, the patient or subject is a primate. Non-limiting examples of human subjects are adults, juveniles, infants and fetuses.

As generally used herein "pharmaceutically acceptable" refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

"Pharmaceutically acceptable salts" means salts of compounds of the present invention which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as 1,2,-ethanedisulfonic acid, 2-hydroxyethanesulfonic acid, 2-naphtalenesulfonic acid, 3-phenylpropionic acid, 4,4'-methylenebis(3-hydroxy-2-ene-1-carboxylic acid), 4-methylbicyclo[2.2.2]oct-2-ene-1-carboxylic acid, acetic acid, aliphatic mono- and dicarboxylic acids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acids, propionic acid, *p*-toluenesulfonic acid, pyruvic acid, salicylic acid, stearic acid, succinic acid, tartaric acid, tertiarybutylacetic acid, trimethylacetic acid, and the like. Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, *N*-methylglucamine and the like. It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts: Properties, and Use (P. H. Stahl & C G. Wermuth eds., Verlag Helvetíca Chimica Acta, 2002).

The term "pharmaceutically acceptable carrier," as used herein means a pharmaceutically-acceptable material, composition or vehicle, such as liquid or solid filler, diluent, excipient, solvent or encapsulating material, involved in carrying or transporting a chemical agent.

"Prevention" or "preventing" includes: (1) inhibiting the onset on a disease in a subject or patient which may be a risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

A "stereoisomer" or "optical isomer" is an isomer of a given compound in which the same atoms are bonded in the same other atoms, but where the configuration of those atoms in three dimensions differs, "Enantiomers" are stereoisomers of given compound that are mirror images of each other, like left and right hands. "Diastereomers" are stereoisomers of a given compound that are not enantiomers. Chiral molecules contain a chiral center, also referred to as a stereocenter or stereogenic center, which is any point, though not necessarily an atom, in a molecule bearing groups such that an interchanging of any two groups leads to a stereoisomer. In organic compounds, the chiral center is typically a carbon, phosphorus or sulfur atom, though it is also possible for other atoms to be stereocenters in organic and inorganic compounds. A molecule can have multiple stereocenters, giving it many stereosomers. In compounds whose stereoisomerism is due to tetrahedral stereogenic centers (*e.g*., tetrahedral carbon), the total number of hypothetically possible stereoisomers will not exceed 2n, where n is the number of tetrahedral stereocenters. Molecules with symmetry frequently have fewer than the maximum possible number of stereoisomers. A 50:50 mixture of enantiomers is referred to as a racemic mixture. Alternatively, a mixture of enantiomers can be enantiomerically enriched so that one enantiomer is present in an amount greater than 50%. Typically, enantiomers and/or diastereomers can be resolved or separated using techniques known in the art. It is contemplated that for any stereocenter or axis of chirality for which stereochemistry has not been defined, that stereocenter or axis of chirality can be present in its *R* form, *S* form, or as a mixture of the *R* and *S* forms, including racemic and non-racemic mixtures. As used herein, the phrase "substantially free from other stereoisomers" means that the composition contains ≤ 15%, more preferably ≤ 10%, even more preferably ≤ 5%, or most preferably ≤ 1% of another stereoisomer(s).

"Treatment" or "treating" includes (1) inhibiting a disease in a subject or patient experiencing or displaying the pathology or symptomatology of the disease (*e.g*., arresting further development of the pathology and/or symptomatology), (2) ameliorating a disease in a subject or patient that is experiencing or displaying the pathology or symptomatology of the disease (*e.g*., reversing the pathology and/or symptomatology), and/or (3) effecting any measurable decrease in a disease in a subject or patient that is experiencing or displaying the pathology or symptomatology of the disease.

Other abbreviations used herein are as follows; DMSO, dimethyl sulfoxide; (COCl)₂, oxalyl chloride; EtN₃ or TEA, triethylamine; DMAP, dimethylaminopyridine; Et₂O, diethyl ether; *n*-PrCONHNH₂, butyric acid hydrazine; *i*-PrCONHNH₂, isobutyric acid hydrazine; *c-*PrCONHNH₂, cyclopropane carboxylic acid hydrazine; *p*-TsOH, *p*-toluenesulfonic acid; DMF, dimethylformamide; EDCl, 1-ethyl-3-(3-dimethylaminopropyl)carbodiimede; NO, nitric oxide; iNOS, inducible nitric oxide synthase; COX-2, cyclooxygenase-2; FBS, fetal bovine serum; IFNγ or IFNγ, interferon-γ; TNFα or TNF-α, tumor necrosis factor-α; IL-1β, interleukin-1β; HO-1, inducible hente oxygenase.

The above definitions supersede any conflicting definition in any of the documents referenced herein. The fact that certain terms are defined, however, should not be considered as indicative that any term that is undefined is indefinite. Rather, all terms used are believed to describe the invention in terms such that one of ordinary skill can appreciate the scope and practice the present invention.

### II. Compounds and Synthetic Methods

The compounds provided the present disclosure are shown above in the summary of the invention, in the claims, and in the sections below. They may be made using the methods outlined in the Examples section. These methods can be further modified and optimized using the and techniques of organic chemistry as applied by a person skilled in the art. Such principles and techniques are taught, for example, in March's Advanced Organic Chemistry: Reactions, Mechanisms, and Structure (2007). Compounds of the invention may contain one or more asymmetrically-substituted carbon or nitrogen atoms, and may be isolated in optically active or racemic form. Thus, all chiral, diastereomeric, racemic form, epimeric form, and all geometric isomeric forms of a chemical formula are intended, unless the specific stereochemistry or isomeric form is specifically indicated. Compounds may occur as racemates and racemic mixtures, single enantiomers, diastereomeric mixtures and individual diastereomers. In some embodiments, a single diastereomer is obtained. The chiral centers of the compounds of the present invention can have the *S* or the *R* configuration.

Chemical formulas used to represent compounds of the invention will typically only show one of possibly several different tautomers. For example, many types of ketone groups are known to exist in equilibrium with corresponding enol groups. Similarly, many types of imine groups exist in equilibrium with enamine groups. Regardless of which tautomer is depicted for a given compound, and regardless of which one is most prevalent, all tautomers of a given chemical formula are intended.

Atoms making up the compounds of the present invention are intended to include all isotopic forms of such atoms. Compounds of the present invention include those with one or more atoms that have been isotopically modified or enriched, in particular those with pharmaceutically acceptable isotopes or those useful for pharmaceutically research. Isotopes, as used herein, include those atoms having the same atomic number but different mass numbers. By way of general example and without limitation, isotopes of hydrogen include deuterium and tritium, and isotopes of carbon include ¹³C and ¹⁴C. Similarly, it is contemplated that one or more carbon atom(s) of a compound of the present invention may be replaced by a silicon atom(s). Furthermore, it is contemplated that one or more oxygen atom(s) of a compound of the present invention may be replaced by a sulfur or selenium atom(s).

It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts: Properties, and Use (2002). It should be further recognized that the compounds of the present invention include those that have been further modified to comprise substituents that are convertible to hydrogen *in vivo.* This includes those groups that may be convertible to hydrogen atom by enzymological or chemical means including, but not limited to, hydrolysis and hydrogenolysis. Examples include hydrolyzable groups, such as acyl groups, groups having an oxycarbonyl group, amino acid residues, peptide residues, *o*-nitrophenylsulfenyl, trimethylsilyl, tetrahydropyranyl, diphenylphosphinyl, and the like. Examples of acyl groups include formyl, acetyl, trifluoroacetyl, and the like. Examples of groups having an oxycarbonyl group include ethoxycarbonyl, *tert*-butoxycarbonyl (-C(O)OC(CH₃)₃, Boc), benzyloxycarbonyl, *p*-methoxybenzyloxycarbonyl, vinyloxycarbonyl, β-(*p-*toluenesulfonyl)ethoxycarbonyl, and the like. Suitable amino acid residues include, but are not limited to, residues of Gly (glycine), Ala (alanine), Arg (arginine), Asn (asparagine), Asp (aspartie acid), Cys (cysteine), Glu (glutamic acid), His (histidine), Ile (isoleucine), Leu (leucine), Lys (lysine), Met (methionine), Phe (phenylalanine), Pro (proline), Ser (serine), Thr (threonine), Trp (tryptophan), Tyr (tyrosine), Val (valine), Nva (norvaline), Hse (homoserine), 4-Hyp (4-hydroxyproline), 5-Hyl (5-hydroxylysine), Orn (ornithine) and β-Ala. Examples of suitable amino acid residues also include amino acid residues that are protected with a protecting group. Examples of suitable protecting groups include those typically employed in peptide synthesis, including acyl groups (such as formyl and acetyl), arylmethoxycarbonyl groups (such as benzyloxycarbonyl and p-nitrobenzyloxycarbonyl), *tert*-butoxycarbonyl groups (-C(O)OC(CH₃)₃, Boc), and the like. Suitable peptide residues include peptide residues comprising two to five amino acid residues. The residues of these amino acids or peptides can be present in stereochemical configurations of the D-form, the L-form or mixtures thereof. In addition, the amino acid or peptide residue may have an asymmetric carbon atom. Examples of suitable amino acid residues having an asymmetric carbon atom include residues of Ala, Leu, Phe, Trp, Nva, Val, Met, Ser, Lys, Thr and Tyr. Peptide residues having an asymmetric carbon atom include peptide residues having one or more constituent amino acid residues having an asymmetric carbon atom. Examples of suitable amino acid protecting groups include those typically employed in peptide synthesis, including acyl groups (such as formyl and acetyl), arylmethoxycarbonyl groups (such as benzyloxycarbonyl and *p*-nitrobenzyloxycarbonyl), *tert*-butoxycarbonyl groups (-C(O)OC(CH₃)₃), and the like. Other examples of substituents "convertible to hydrogen *in vivo*" include seductively eliminable hydrogenolizable groups. Examples of suitable reductively eliminable hydrogenolyzable groups include, but are not limited to, arylsulfonyl groups (such as *o*-toluenesulfonyl); methyl groups substituted with phenyl or benzyloxy (such as benzyl, trityl and benzyloxymethyl); arymethoxycarbonyl groups (such as benzyloxycarbonyl and o-methoxy-benzyloxycarbonyl); and haloethoxycarbonyl groups (such as β,β,β-trichloroethoxycarbonyl and β-iodoethoxycarbonyl).

Compounds of the invention may also have the advantage that they may be more efficacious than, be less toxic than, be longer acting than, be more potent than, produce fewer side effects than, be more easily absorbed than, and/or have a better pharmacokinetic profile (*e.g*., higher oral bioavailability and/or lower clearance) than, and/or have other useful pharmcological, physical, or chemical properties over, compounds known in the prior art, whether for use in the indications stated herein or otherwise.

### III. Biological Activity

Assay results for the suppression of IFNγ-induced NO production are shown for several of the compounds of the present invention in. Table 1 below. In the right-hand column of this table under the RAW264.7 heading, the results are compared to those of bardoxolone methyl (RTA 402, CDDO-Me). Details regarding this assay are provided in the Examples section below.

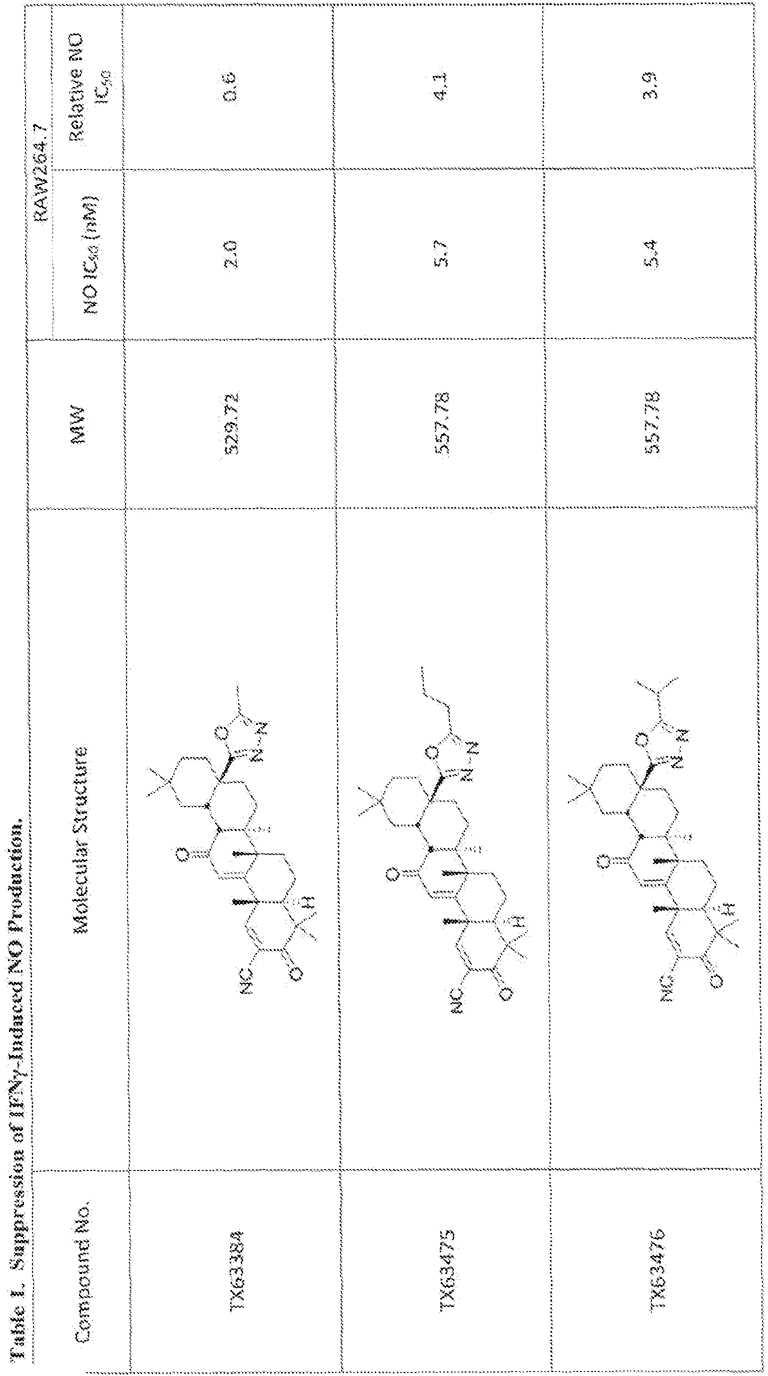

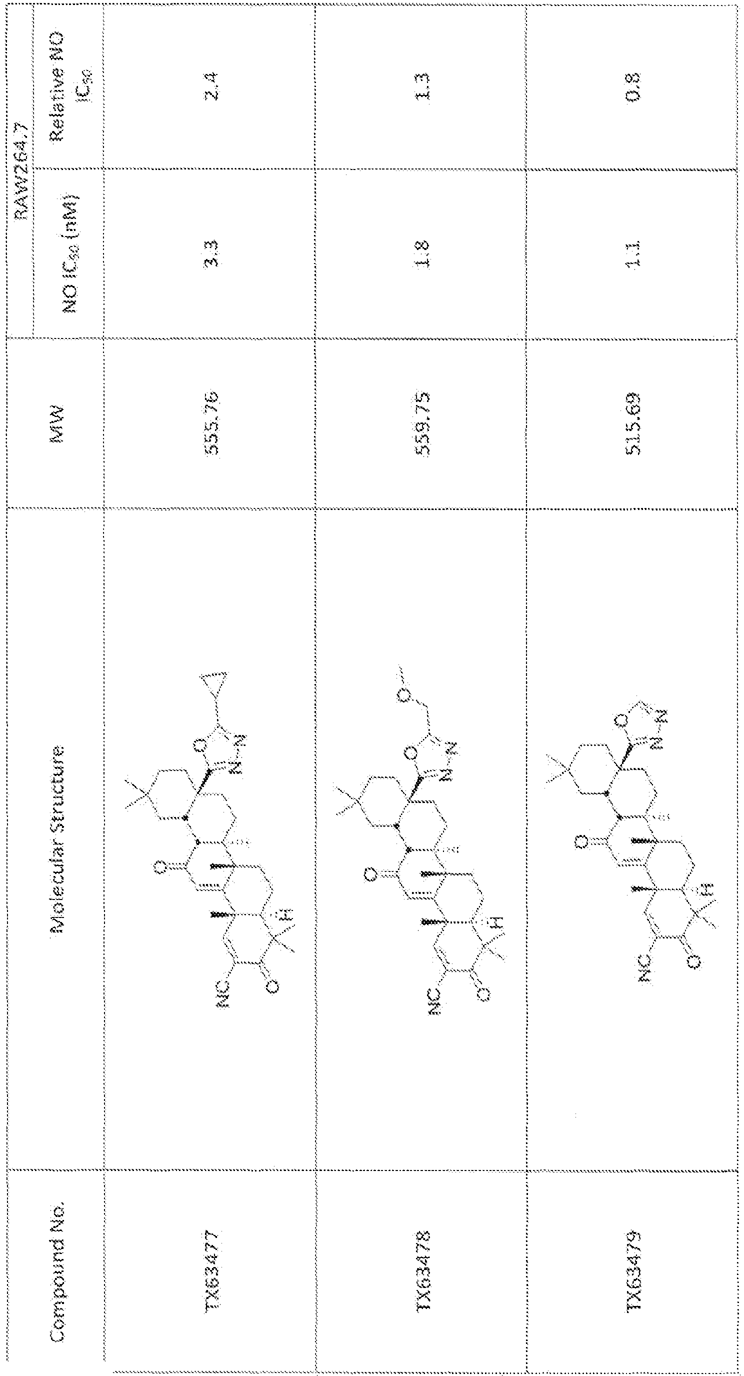

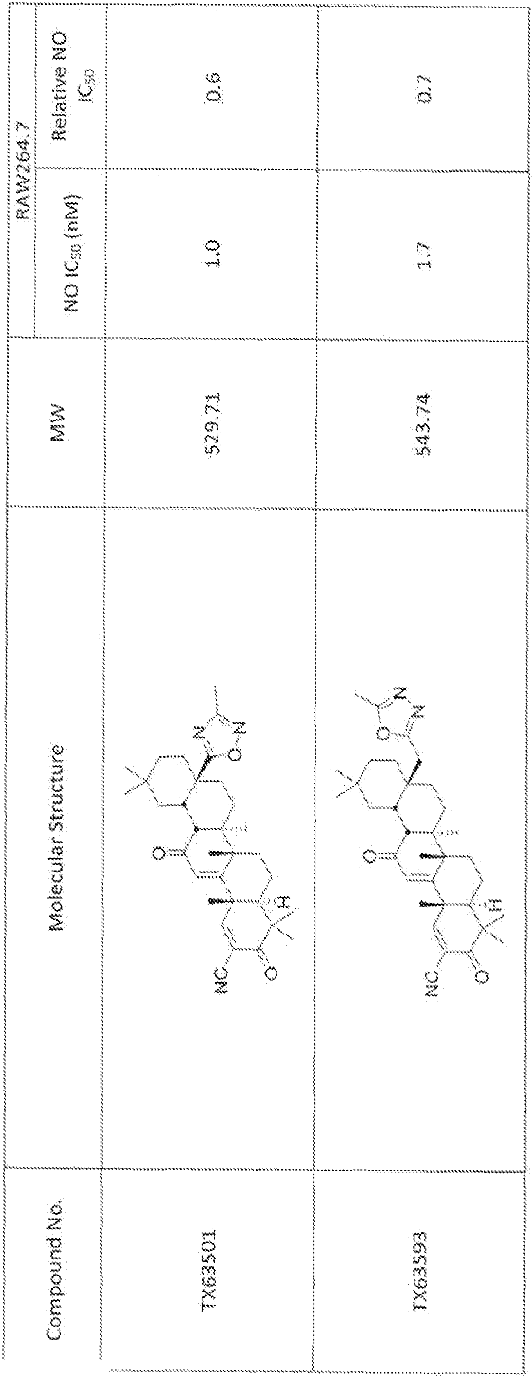

### IV. Diseases Associated with Inflammation and/or Oxidative Stress

Inflammation is a biological process that provides resistance to infectious or parasitic organisms and the repair of damaged tissue. Inflammation is commonly characterized by localized vasodilation, redness, swelling, and pain, the recruitment of leukocytes to the site of infection or injury, production of inflammatory cytokines such as TNF-α and IL-1, and production of reactive oxygen or nitrogen species such as hydrogen peroxide, superoxide and peroxynitrite. In later stages of inflammation, tissue remodeling, angiogenesis, and scar formation (fibrosis) may occur as part of the wound healing process. Under normal circumstances, the inflammatory response is regulated and temporary and is resolved in an orchestrated fashion once the infection or injury has been dealt with adequately. However, acute inflammation can become excessive and life-threatening if regulatory mechanisms fail. Alternatively, inflammation can become chronic and cause cumulative tissue damage or systemic complications. Based at least on the evidence presented above, the compounds of this invention may be used in the treatment or prevention of inflammation or diseases associated with inflammation.

Many serious and intractable human diseases involve dysregulation of inflammatory processes, including diseases such as cancer, atherosclerosis, and diabetes, which were not traditionally viewed as inflammatory conditions. In the case of cancer, the inflammatory processes are associated with tumor formation, progression, metastasis, and resistance to therapy. Atherosclerosis, long viewed as a disorder of lipid metabolism, is now understood to be primarily an inflammatory condition, with activated macrophages playing an important role in the formation and eventual rupture of atherosclerotic plaques. Activation of inflammatory signaling pathways has also been shown to play a role in the development of insulin resistance, as well as in the peripheral tissue damage associated with diabetic hyperglycemia. Excessive production of reactive oxygen species and reactive nitrogen species such as superoxide, hydrogen peroxide, nitric oxide, and peroxynitrite is a hallmark of inflammatory conditions. Evidence of dysregulated peroxynitrite production has been reported in a wide variety of diseases (Szabo *et al*., 2007; Schulz *et al*., 2008; Forstermann, 2006; Pall, 2007).

Autoimmune diseases such as rheumatoid arthritis, lupus, psoriasis, and multiple sclerosis involve inappropriate and chronic activation of inflammatory processes in affected tissues, arising from dysfunction of self vs. non-self recognition and response mechanisms in the immune system. In neurodegenerative diseases such as Alzheimer's and Parkinson's diseases, neural damage is correlated with activation of microglia and elevated levels of pro-inflammatory proteins such as inducible nitric oxide synthase (iNOS). Chronic organ failure such as renal failure, heart failure, liver failure, and chronic obstructive pulmonary disease is closely associated with the presence of chronic oxidative stress and inflammation, leading to the development of fibrosis and eventual loss of organ function. Oxidative stress in vascular endothelial cells, which line major and minor blood vessels, can lead to endothelial dysfunction and is believed to be an important contributing factor in the development of systemic cardiovascular disease, complications of diabetes, chronic kidney disease and other forms of organ failure, and a number of other aging-related diseases including degenerative diseases of the central nervous system and the retina.

Many other disorders involve oxidative stress and inflammation in affected tissues, including inflammatory bowel disease; inflammatory skin diseases; mucositis related to radiation therapy and chemotherapy; eye diseases such as uveitis, glaucoma, macular degeneration, and various forms of retinopathy; transplant failure and rejection; ischemia-reperfusion injury; chronic pain; degenerative conditions of the bones and joints including osteoarthritis and osteoporosis; asthma and cystic fibrosis; seizure disorders; and neuropsychiatric conditions including schizophrenia, depression, bipolar disorder, post-traumatic stress disorder, attention deficit disorders, autism-spectrum disorders, and eating disorders such as anorexia nervosa. Dysregulation of inflammatory signaling pathways is believed to be a major factor in the pathology of muscle wasting diseases including muscular dystrophy and various forms of cachexia.

A variety of life-threatening acute disorders also involve deregulated inflammatory signaling, including acute organ failure involving the pancreas, kidneys, liver, or lungs, myocardial infarction or acute coronary syndrome, stroke, septic shock, trauma, severe burns, and anaphylaxis.

Many complications of infectious diseases also involve dysregulation of inflammatory responses. Although an inflammatory response can kill invading pathogens, an excessive inflammatory response can also be quite destructive and in some cases can be a primary source of damage in infected tissues. Furthermore, an excessive inflammatory response can also lead to systemic complications due to overproduction of inflammatory cytokines such as TNF-α and IL-1. This is believed to be a factor in mortality arising from severe influenza, severe acute respiratory syndroms, and sepsis.

The aberrant or excessive expression of either iNOS or cyclooxygenase-2 (COX-2) has been implicated in the pathogenesis of many disease processes. For example, it is clear that NO is a potent mutagen (Tamir and Tannebaum, 1996), and that nitric oxide can also activate COX-2 (Salvemini *et al*., 1994). Furthermore, there is a marked increase in iNOS in rat colon tumors induced by the carcinogen, azoxymethane (Takahashi *et al*., 1997). A series of synthetic triterpenoid analogs of oleanolic acid have been shown to be powerful inhibitors of cellular inflammatory processes, such as the induction by IFN-γ of inducible nitric oxide synthase (iNOS) and of COX-2 in mouse macrophages. See Honda *et al*. (2000a); Honda *et al*. (2002b), and Honda *et al*. (2002). In one aspect, compounds disclosed herein are characterized by their ability to inhibit the production of nitric oxide in macrophage-derived RAW 264,7 cells induced by exposure to γ-interferon. They are further characterized by their ability to induce the expression of antioxidant proteins such as NQO1 and reduce the expression of pro-inflammatory proteins such as COX-2 and inducible nitric oxide synthase (iNOS). These properties are relevant to the treatment of a wide array of diseases and disorders involving oxidative stress and dysregulation of inflammatory processes including cancer, complications from localized or total-body exposure to ionizing radiation, mucositis resulting from radiation therapy or chemotherapy, autoimmune diseases, cardiovascular diseases including atherosclerosis, ischemia-reperfusion injury, acute and chronic organ failure including renal failure and heart failure, respiratory diseases, diabetes and complications of diabetes, severe allergies, transplant rejection, graft-versus-host disease, neurodegenerative diseases, diseases of the eye and retina, acute and chronic pain, degenerative bone diseases including osteoarthritis and osteoporosis, inflammatory bowel diseases, dermatitis and other skin diseases, sepsis, burns, seizure disorders, and neuropsychiatric disorders.

Without being bound by theory, the activation of the antioxidant/anti-inflammatory Keap1/Nrf2/ARE pathway is believed to be implicated in both the anti-inflammatory and anti-carcinogenic properties of the compounds disclosed herein.

In another aspect, compounds disclosed herein may be used for treating a subject having a condition caused by elevated levels of oxidative stress in one or more tissues. Oxidative stress results from abnormally high or prolonged levels of reactive oxygen species such as superoxide, hydrogen peroxide, nitric oxide, and peroxynitrite (formed by the reaction of nitric oxide and superoxide). The oxidative stress may be accompanied by either acute or chronic inflammation. The oxidative stress may be caused by mitochondrial dysfunction, by activation of immune cells such as macrophages and neutrophils, by acute exposure to an external agent such as ionizing radiation or a cytotoxic chemotherapy agent (*e.g*., doxombicin), by trauma or other acute tissue injury, by ischemia/reperfusion, by poor circulation or anemia, by localized or systemic hypoxia or hyperoxia, by elevated levels of inflammatory cytokines and other inflammation-related proteins, and/or by other abnormal physiological states such as hyperglycemia or hypoglycemia.

In animal models of many such conditions, stimulating expression of inducible heme oxygenase (HO-1), a target gene of the Nrf2 pathway, has been shown to have a significant therapeutic effect including models of myocardial infarction, renal failure, transplant failure and rejection, stroke, cardiovascular disease, and autoimmune disease (*e.g*., Sacerdoti *et al*., 2005; Abraham & Kappas, 2005; Bach, 2006; Araujo *et al*., 2003; Liu *et al.*, 2006; Ishikawa *et al.*, 2001; Kroger *et al.,* 2006; Satoh *et al.*, 2006; Zhou *et al.*, 2005; Morse and Choi, 2005; Morse and Choi, 2002). This enzyme breaks free heme down into iron, carbon monoxide (CO), and biliverdin (which is subsequently converted to the potent antioxidant molecule, bilirubin).

In another aspect, compounds of this invention may be used in preventing or treating tissue damage or organ failure, acute and chronic, resulting from oxidative stress exacerbated by inflammation. Examples of diseases that fall in this category include: heart failure, liver failure, transplant failure and rejection, renal failure, pancreatitis, fibrotic lung diseases (cystic fibrosis, COPD, and idiopathic pulmonary fibrosis, among others), diabetes (including complications), atherosclerosis, ischemia-reperfusion injury, glaucoma, stroke, autoimmune disease, autism, macular degeneration, and muscular dystrophy. For example, in the case of autism, studies suggest that increased oxidative stress in the central nervous system may contribute to the development of the disease (Chauhan and Chauhan, 2006).

Evidence also links oxidative stress and inflammation to the development and pathology of many other disorders of the central nervous system, including psychiatric disorders such as psychosis, major depression, and bipolar disorder; seizure disorders such as epilepsy; pain and sensory syndromes such as migraine, neuropathic pain or tinnitus; and behavioral syndromes such as the attention deficit disorders. See, *e.g*., Dickerson *et al*., 2007; Hanson *et al.*, 2005; Kendall-Tackett, 2007; Lenez *et al*., 2007; Dudhgaonkar *et al*., 2006; Lee *et al*., 2007; Mortis *et al*., 2002; Ruster *et al*., 2005; McIver *et al.,* 2005; Sarchielli *et al*., 2006; Kawakami *et al*., 2006; Ross *et al*., 2003. For example, elevated levels of inflammatory cytokines, including TNF, interferon-γ, and IL-6, are associated with major mental illness (Dickerson *et al.,* 2007), Microglial activation has also been linked to major mental illness. Therefore, downregulating inflammatory cytokines and inhibiting excessive activation of microglia could be beneficial in patients with schizophrenia, major depression, bipolar disorder, autism-spectrum disorders, and other neuropsychiatric disorders.

Accordingly, in pathologies involving oxidative stress alone or oxidative stress exacerbated by inflammation, treatment may comprise administering to a subject a therapeutically effective amount of a compound of this invention, such as those described above or throughout this specification. Treatment may be administered preventively, in advance of a predictable state of oxidative stress (*e.g*., organ, transplantation or the administration of radiation therapy to a cancer patient), or it may be administered therapeutically to settings involving established oxidative stress and inflammation.

The compounds disclosed herein may be generally applied to the treatment of inflammatory conditions, such as sepsis, dermatitis, autoimmune disease and osteoarthritis. In one aspect, the compounds of this invention may be used to treat inflammatory pain and/or neuropathic pain, for example, by inducing Nrf2 and/or inhibiting NF-κB.

In some the compounds disclosed herein may be used in the treatment and prevention of diseases such as cancer, inflammation, Alzheimer's disease, Parkissson's disease, multiple sclerosis, autism, amyotrophic lateral sclerosis, Huntington's disease, autoimmune diseases such as rheumatoid arthritis, lupus, Crohn's disease and psoriasis, inflammatory bowel disease, all other diseases whose pathogenesis is believed to involve excessive production of either nitric oxide or prostaglandins, and pathologies involving oxidative stress alone or oxidative stress exacerbated by inflammation.

Another aspect of inflammation is the production of inflammatory prostaglandins such as prostaglandin E. These molecules promote vasodilation, plasma extravasation, localized pain, elevated temperature, and other symptoms of inflammation. The inducible form of the enzyme COX-2 is associated with their production, and high levels of COX-2 are found in inflamed tissues. Consequently, inhibition of COX-2 may relieve many symptoms of inflammation and a number of important anti-inflammatory drugs (*e.g*., ibuprofen and celecoxib) act by inhibiting COX-2 activity. Recent research, however, has demonstrated that a class of cyclopentenone prostaglandins (cyPGs) (*e.g*., 15-deoxy prostaglandin J2, a.k.a. PGJ2) plays a role in stimulating the orchestrated resolution of inflammation (*e.g*., Rajakariar *et al.,* 2007). COX-2 is also associated with the production of cyclopentenone prostaglandins. Consequently, inhibition of COX-2 may interfere with the full resolution of inflammation, potentially promoting the persistence of activated immune cells in tissues and leading to chronic, "smoldering" inflammation. This, effect may be responsible for the increased incidence of cardiovascular disease in patients using selective COX-2 inhibitors for long periods of time.

In one aspect, the compounds disclosed herein may be used to control the production of pro-inflammatory cytokines within the cell by selectively activating regulatory cysteine residues (RCRs) on proteins that regulate the activity of redox-sensitive transcription factors. Activation, of RCRs by cyPGs has been shown to initiate a pro-resolution program in which the activity of the antioxidant, and cytoprotective transcription factor Nrf2 is potently induced and the activities of the pro-oxidant and pro-inflammatory transcription factors NF-κB and the STATs are suppressed. In some embodiments, this increases the production of antioxidant and reductive molecules (NQO1, HO-1, SOD1, γ-GCS) and decreases oxidative stress and the production of pro-oxidant and pro-inflammatory molecules (iNOS, COX-2, TNF-α). In some embodiments, the compounds of this invention may cause the cells that host the inflammatory event to revert to a non-inflammatory state by promoting the resolution of inflammation and limiting excessive tissue damage to the host.

### V. Pharmaceutical Fomulations and Routes of Administration

The compounds of the present disclosure may be administered by a variety of methods, *e.g*., orally or by injection (*e.g*. subcutaneous, intravenous, intraperitoneal *etc*.). Depending on the route of administration, the active compounds may be coated in a material to protect the compound from the action of acids and other natural conditions which may inactivate the compound. They may also be administered by continuous perfusion/infusion of a disease or wound site.

To administer the therapeutic compound by other than parenteral administration, it may be necessary to coat the compound with, or co-administer the compound with, a material to prevent its inactivation. For example, the therapeutic compound may be administered to a patient in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan *et al*., 1984).

The therapeutic compound may also be administered parenterally, intraperitoneally, intraspinally, or intracerebrally. Dispersions can be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include: sterile aqueous solutions (where water soluble), dispersions, and sterile powders for the extemporaneous preparation of sterile injectable solutions or dispersion. See for example U.S. Patent Application by J. Zhang, entitled "Amorphous Solid Dispersions of CDDO-Me for Delayed Release Oral Dosage Compositions." filed. February 13, 2009. In all cases, the composition must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (such as, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, sodium chloride, or polyalcohols such as mannitol and sorbitol, in the composition. Prolonged absorption of the injectable compositions can be brought about by including in the composition an agent which delays absorption, for example, aluminum monostsarate or gelatin.

Sterile injectable solutions can be prepared by incorporating the therapeutic compound in the required amount in an appropriate solvent with one or a combination of ingredients enumerated above, as required, followed by filtered sterilization. Generally, dispersions are prepared by incorporating the therapeutic compound into a sterile carrier which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (*i.e*., the therapeutic compound) plus any additional desired ingredient from a previously sterile-filtered solution thereof.

The therapeutic compound can be orally administered, for example, with an inert diluent or an assimilable edible carrier. The therapeutic compound and other ingredients may also be enclosed in a hard or soft shell gelatin capsule, compressed into tablets, or incorporated directly into the subject's diet. For oral therapeutic administration, the therapeutic compound may be incorporated with excipients and used in the form of ingestible tablets, buccal tablets, troches, capsules, elixirs, suspensions, syrups, wafers, and the like. The percentage of the therapeutic compound in the compositions and preparations may, of course, be varied. The amount of the therapeutic compound in such therapeutically useful composition is such that a suitable dosage will be obtained.

It is especially advantageous to formulate parenteral compositions in dosage unit form for ease of admimstration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical carrier. The specification for the dosage unit forms of the invention are dictated by and directly dependent on (a) the unique characteristics of the therapeutic compound and the particular therapeutic effect to be achieved, and (b) the limitations inherent in the an of compounding such a therapeutic compound for the treatment of a selected condition in a patient.

The therapeutic compound may also be administered to to the skin, eye, or mucosa. Alternatively, if local delivery to the lungs is desired the therapeutic compound may be administered by inhalation in a dry-powder or aerosol formulation.

Active compounds are administered at a a therapeutically effective dosage sufficient to treat a condition associated with a condition in a patient. For example, the efficacy of a compound can be evaluated in an animal model system that may be predictive of efficacy in treating the disease in humans, such as the model systems shown in the examples and drawings.

The actual dosage amount of a compound of the present disclosure or composition comprising a compound of the present disclosure administered to a subject may be determined by physical and physiological factors such as age, sex, body weight, severity of condition, the type of disease being treated, previous or concurrent therapeutic interventions. idiopathy of the subject and on the mute of administration. These factors may be determined by a skilled artisan. The practitioner responsible for administration will typically determine the concentration of active ingredient(s) in a composition and appropriate dose(s) for the individual, subject. The dosage may be adjusted by the individual physician in the event of any complication.

An effective amount typically will vary from about 0.001 mg/kg to about 1000 mg/kg, from about 0.01 mg/kg to about 750 mg/kg, from about 100 mg/kg to about 500 mg/kg, from about 1.0 mg/kg to about 250 mg/kg, from about 10.0 mg/kg to about 150 mg/kg in one or more dose administrations daily, for one or several days (depending of course of the mode of administration and the factors discussed above). Other suitable dose ranges include 1 mg to 10000 mg per day, 100 mg to 10000 mg per day, 500 mg to 10000 mg per day, and 500 mg to 1000 mg per day. In some particular embodiments, the amount is less than 10,000 mg per day with a range of 750 mg to 9000 mg per day.

The effective amount may be less than 1 mg/kg/day, less than 500 mg/kg/day, less than 250 mg/kg/day, less than 100 mg/kg/day, less than 50 mg/kg/day, less than 25 mg/kg/day or less than 10 mg/kg/day. It may alternatively be in the range of 1 mg/kg/day to 200 mg/kg/day. For example, regarding treatment of diabetic patients, the unit dosage may be an amount that reduces blood glucose by at least 40% as compared to an untreated subject. In another embodiment, the unit dosage is an amount that reduces blood glucose to a level that is ± 10% of the blood glucose level of a non-diabetic subject.

In other non-limiting examples, a dose may also comprise from about 1 microgram/kg/body weight, about 5 microgram/kg/body weight, about 10 microgm/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, *etc*., can be administered, based on the numbers described above.

In certain embodiments, a pharmaceutical composition of the present disclosure may comprise, for example, at least about 0.1% of a compound of the present disclosure. In other embodiments, the compound of the present disclosure may comprise between about 2% to about 75% of the weight of the unit, or between about 25% to about 60%, for example, and any range derivable therein.

Single or multiple doses of the agents are contemplated. Desired time intervals for delivery of multiple doses can be determined by one of ordinary skill in the art employing no more than routine experimentation. As an example, subjects may be administered two doses daily at approximately 12 hour intervals. In some embodiments, the agent is administered once a day.

The agent(s) may be administered on a routine schedule. As used herein a routine schedule refers to a predetermined designated period of time. The routine schedule may encompass periods of time which are identical or which differ in length, as long as the schedule is predetermined. For instance, the routine schedule may is involve administration twice a day, every day, every two days, every three days, every four days, every five days, every six dsys, a weekly basis, a monthly basis or any set number of days or weeks there-betwen. Alternatively, the predetermined routine schedule may involve administration on a twice daily basis for the first week, followed by a daily basis for several months, *etc*. In other embodiments, the invention provides that the agent(s) may be taken orally and that the timing of which is or is not dependent upon food intake. Thus, for example the agent can be taken every morning and/or every evening, regardless of when the subject has eaten or will eat.

### VI. Combination Therapy

In addition to being used as a monotherapy, the compounds of the present invention may also find use in combination therapies. Effective combination therapy may be achieved with a single composition or pharmacological formulation that includes both agents, or with two distinct compositions or formulations, administered at the same time, wherein one composition inclndes a compound of this invention, and the other includes the second agent(s). Alternatively, the therapy may precede or follow the other agent treatment by intervals ranging from minutes to months.

Non-limiting examples of such combination therapy include combination of one or more compounds of the invention with another anti-inflammatory agent, a chemotherapeutic agent, radiation, therapy, an antidepressant, an antipsychotic agent, an anticonvulsant, a mood stabiliser, an anti-infective agent, an antihypertensive agent, a cholesterol-lowering agent or other modulator of blood lipids, an agent for promoting weight loss, an antithrombotic agent, an agent for treating or preventing cardiovascular events such as myocardial infarction or stroke, an antidiabetic, agent, an agent for reducing transplant rejection or graft-versus-host disease, an anti-arthritic agent, an analgesic agent, an anti-asthmatic agent or other treatment for respiratory diseases, or an agent for treatment or prevention of skin disorders. Compounds of the invention may be combined with agents designed to improve a patient's immune response to cancer, including (but not limited to) cancer vaccines. See Lu *et al.* (2011).

### VII. Examples

The following examples are included to demonstrate preferred embodiments of the invention. It should be appreciated by those of skill in the art that the techniques disclosed in the example which follow represent techniques discovered by the inventor to function well in the practice of the invention, and thus can be considered to constitute preferred modes for its practice.

### Methods and Materials

**Nitric Oxide Production and Cell Viability Assay.** RAW264.7 mouse macrophages were plated in 96-well plates at 30,000 cells/well in triplicate In RPMI1640 + 0.5% FBS and incubated at 37°C with 5% CO₂. On the next day, cells were pre-treated with DMSO or drug (0-200 nM dose range) for 2 hours, and then treated with recombinant mouse IFNγ (R&D Systems) for 24 hours. Nitric Oxide concentration in media was determined using the Griess reagent system (Promega). Cell viability was determined using WST-1 reagent (Roche). IC₅₀ values were determined based on the suppression of IFNγ induced Nitric Oxide production normalized to cell viability.

**NQO1-ARE Luciferase Reporter Assay.** This assay allows for quantitative assessment of the endogenous activity of the Nrf2 transcription factor in cultured mammalian cells. Expression of *Firefly* luciferase from NQO1-ARE luciferase reporter plasmid is controlled by binding of Nrf2 to a specific enhancer sequence corresponding to the antioxidant response clement (ARE) that was identified in the promoter region of the human NADFH:qiunone oxidoredaclase 1 (*NQO1*) gene (Xie *et al.*, 1995). The plasmid was constructed by inserting a sequence:
5'-CAGTCACAGTGACTCAGGAGAATCTG-3' (SEQ ID NO:1) encompassing the human NQO1-ARE into the pLuc-MCS vector using HindIII/XhoI cloning sites (GenScript Corp., Piscataway, NJ). The assay is performed is HuH7 cells maintained in DMEM (Invitrogen) supplemented with 10% FBS and 100U/ml (each) of penicillin and streptomycin. For the assay, cells are plated in 96-well plates at 17.000 cells per well. Twenty four hours later, the cells are co-transfected with 50 ng each of NQO1-ARE reporter plasmid and pRL-TK plasmid using Lipofectamine 2000 transfection reagent (invitrogen). pRL-TK plasmid constitutively expresses *Renilla* luciferase and is used as an internal control for normalization of transfection levels. Thirty hours after transfection, the cells are treated with compounds (at conceatrations ranging from 0 to 1 µM) for eighteen hours. *Firefly* and *Renilla* luciferase activity is assayed by Dual-Glo Laciferase. Assay (Promega Corp., Madison, WI), the luminescence signal is measured on an L-Max II luminemeter (Molecular Devices). *Firefly* luciferase activity is normalized to the *Renilla* activity, and fold induction over a vehicle control (DMSO) of normalized *Firefly* activity is calculated. The fold anduction at 62.3 nM concentration is used for comparing relative potencies of compounds to induce Nrf2 transcriptional activity. Ses Xie *et al.,* 1995.

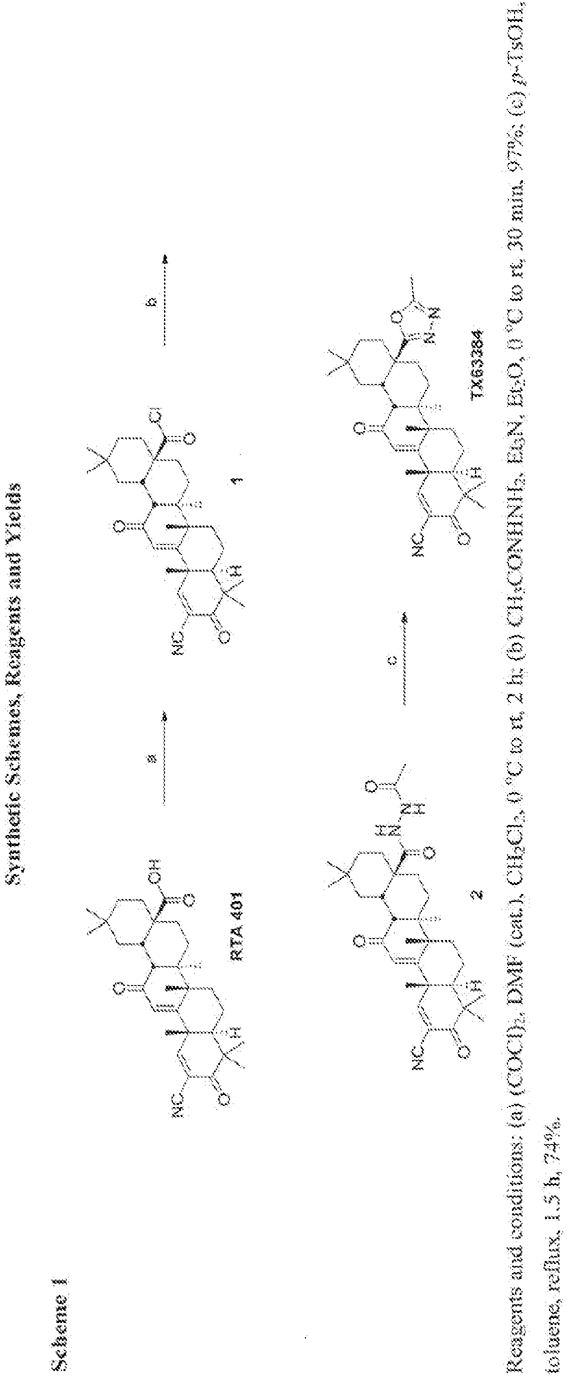

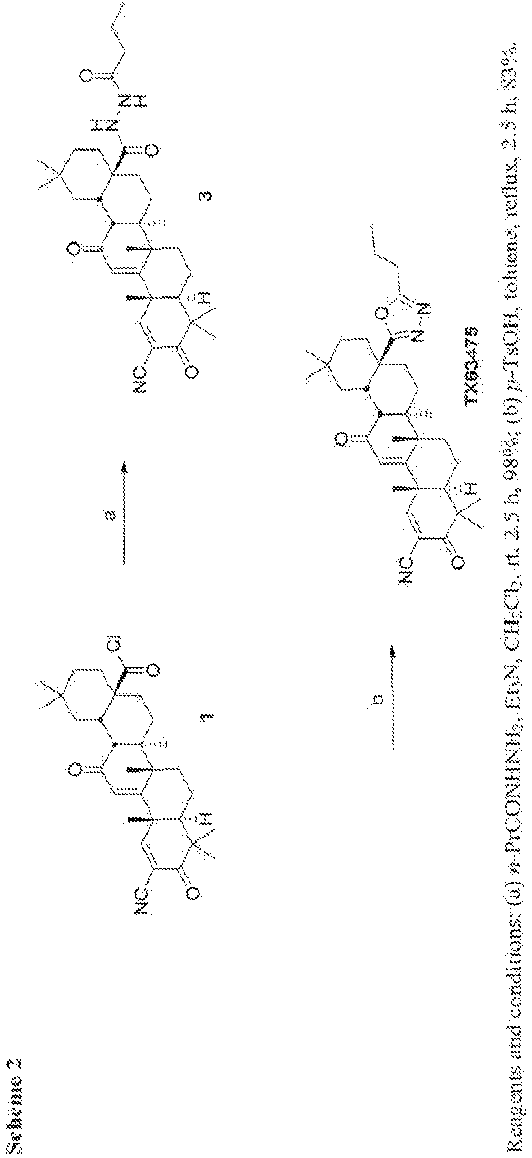

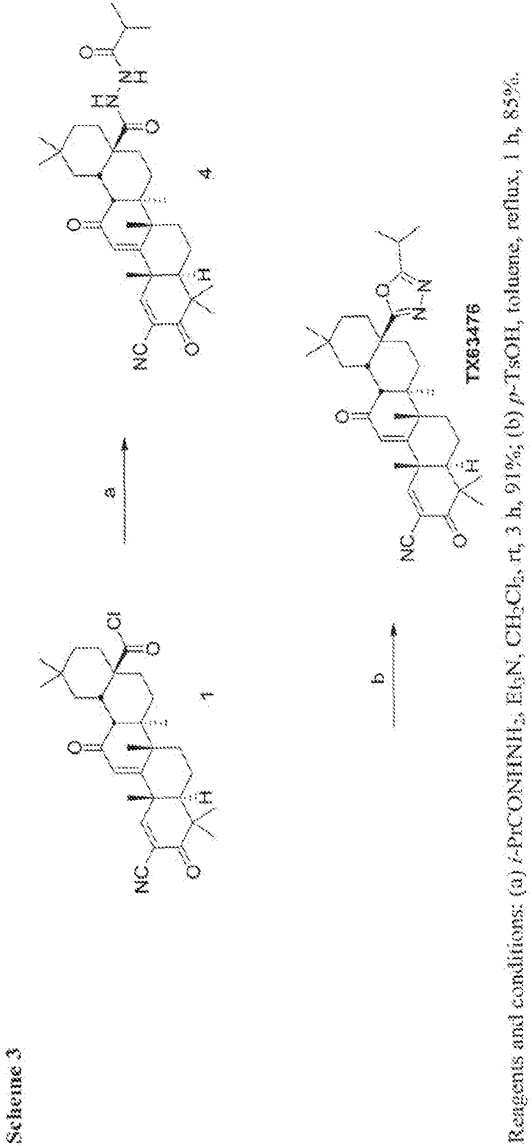

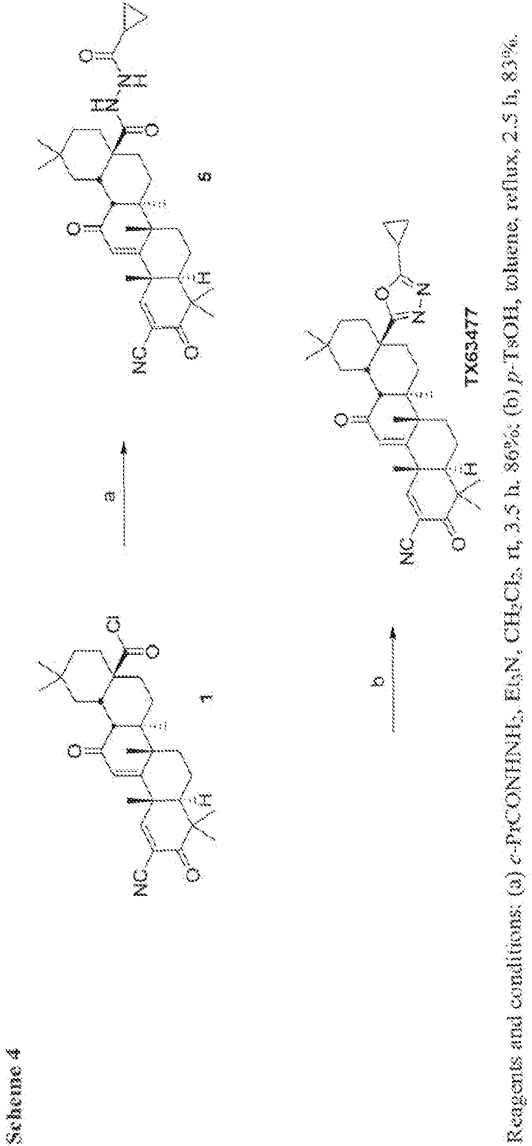

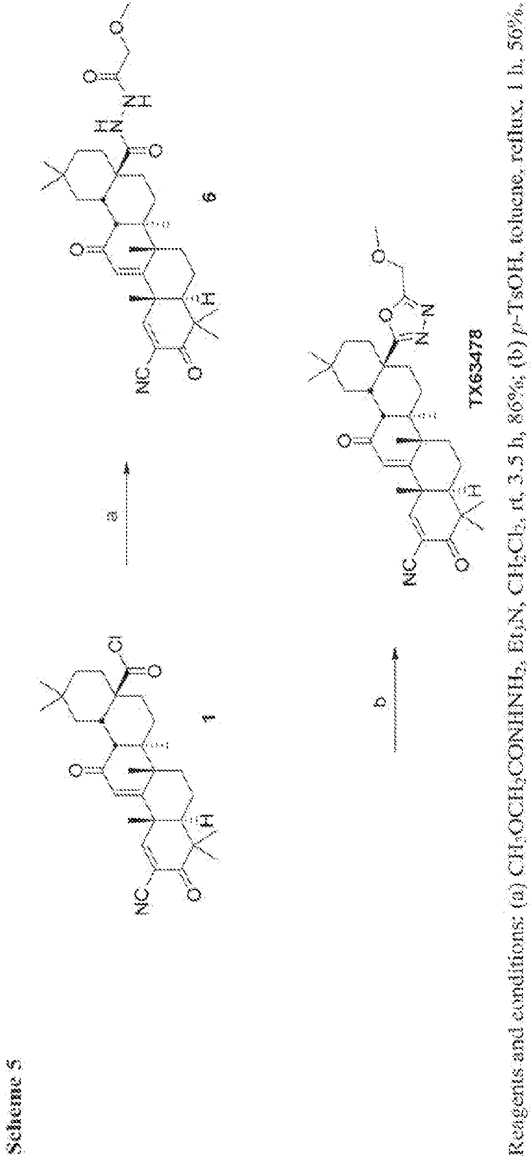

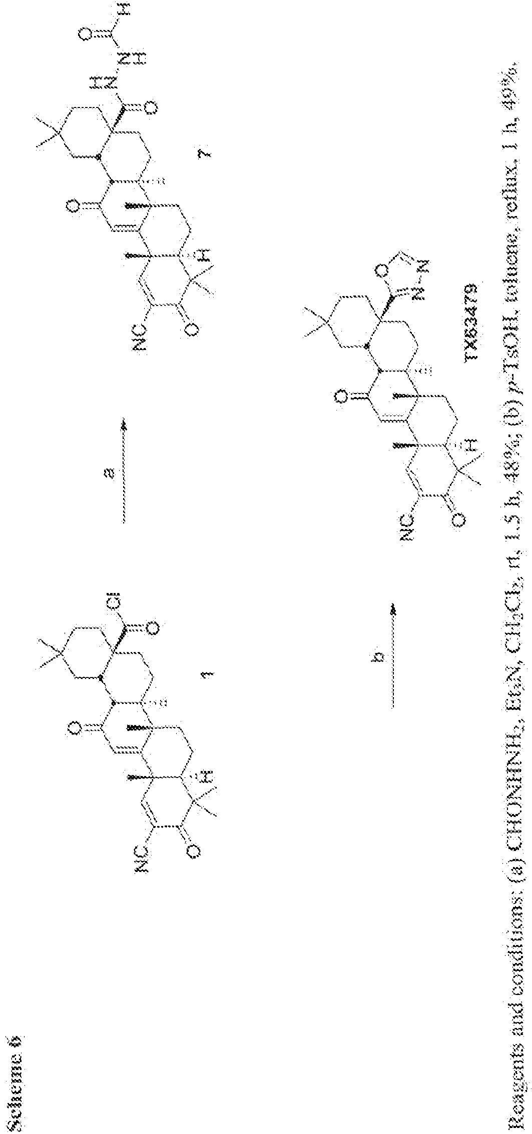

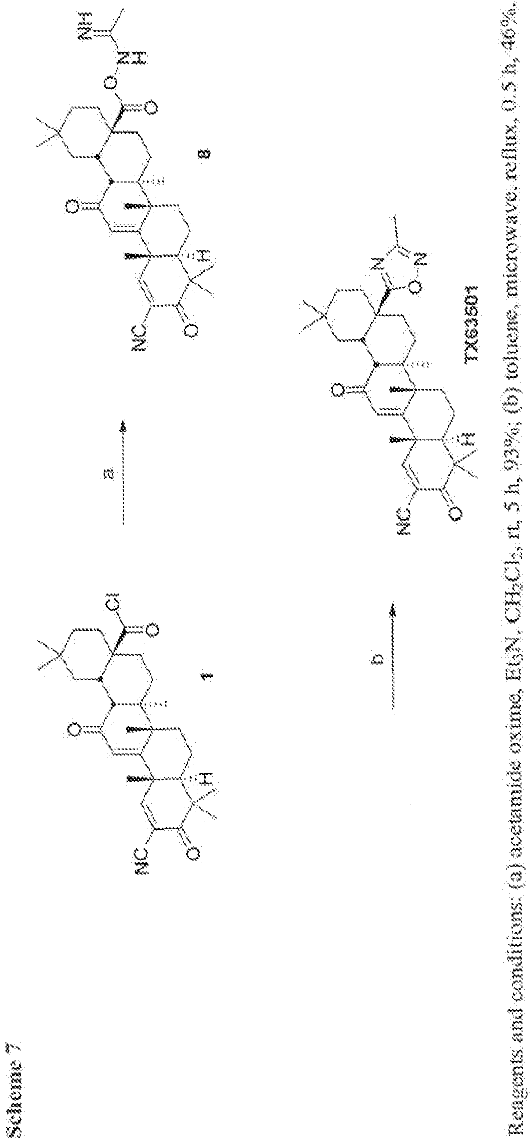

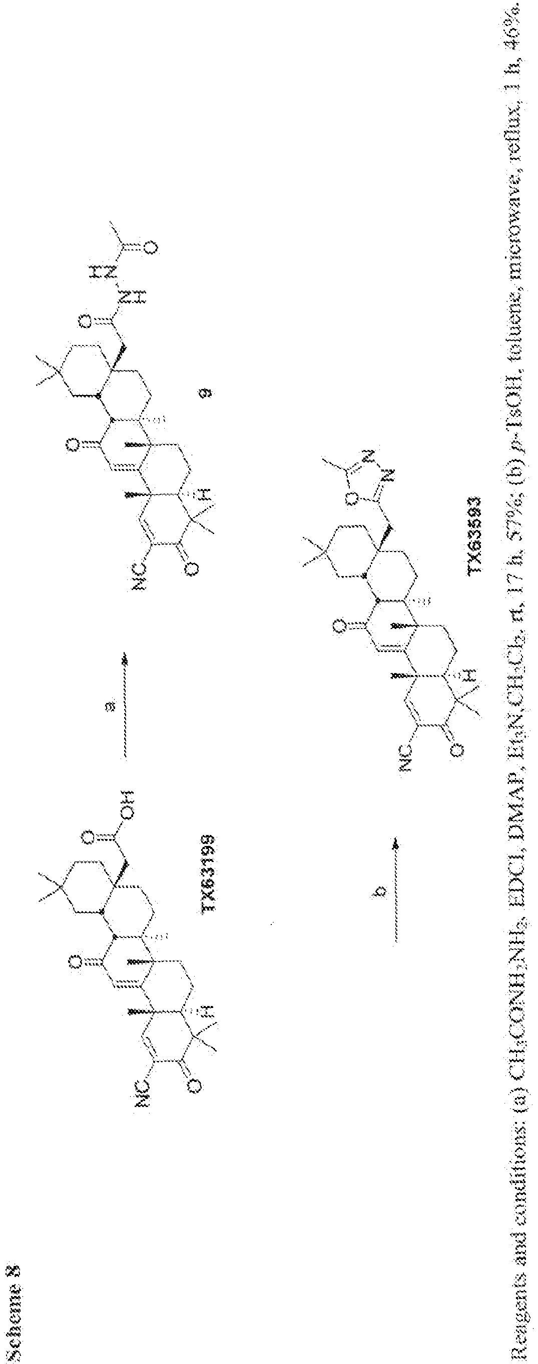

### Synthesis and Characterization of Compounds and Intermediates

**Compound 1:** Compound **RTA 401** (1.00 g, 2.03 mmol) was-dissolved in CH₂Cl₂ (20 mL), and the solution was cooled to 0 °C. Oxalyl chloride (0.55 mL, 6.50 mmol) was added, followed by DMF (2 drops). The reaction mixture was stirred at room temperature for 2 h, then the reaction mixture was concentrated. The residue was azeotroped 2 with CH₂Cl₂ to give compound **1** as a yellow foam, which was used directly in the next step.
**Compound 2:** Compound **1** (2.03 mmol) was dissolved in Et₂O (20 mL), and the solution was cooled to 0 °C To the reaction mixture were added Et₃N (0.565 mL, 4.05 mmoL) and a solution of acethydrazide (226 mg, 3,05 mmol) in CH₂Cl₂ (10 mL). The reaction mixture was stirred at room temperature for 30 min, and was then extracted with EtOAc and washed with water, 1 N HCl, and water again. The organic extracts were dried over MgSO₄, filtered, and concentrated. The residue was purified by flash chromatography (silica gel, 0% to 100% EtOAc in hexanes) to give compound **2** (1.08 g, 97% from **RTA 401**) as an off-white foam solid: m/z 548.3 (M+1).
**Compound TX63384**: To a solution of compound **2** (548 mg, 1.00 mmol) in toluene (20 mL) was added *p*-TsOH (95 mg, 0.50 mmol). The reaction was heated at 135 °C with a Dean-Stark condenser attached for 1.5 h. After cooling to room temperature, the reaction mixture was washed with water, dried over MgSO₄, filtered, and concentrated. The residue was purified by flash chromatography (silica gel, 0% to 70% EtOAc in hexanes) to give compound **TX63384** (390 mg, 74%) as an off-white foam solid: ¹H NMR (400 MHz, CDCl₃) δ 8.02 (s, 1H), 5,96 (s, 1H), 3.13 (m, 1H) 2.94 (d, 1H, *J* = 4.5 Hz), 2.53 (s, 3H), 2.19 (m, 1H), 1.20-2.05 (m, 1H), 1.45 (s, 3H), 1.25 (s, 3H), 1.19 (s, 3H), 1.16 (s, 3H), 1.00 (s, 3H), 1.05 (s, 3H), 0.95 (s, 3H), m/z 530.3 (M+1).
**Compound 3:** To a solution of butyric acid hydrazide (156 mg, 1.53 mmol) and Et₃N (0.58 mL, 4.16 mmol) in CH₂Cl₂ (5 mL) was added a solution of compound **1** (510 mg, 1.00 mmol) in CH₂Cl₂ (5.0 mL). The reaction mixture was stirred at room temperature for 2.5 h. The reaction mixture was then extracted with EtOAc and washed with 1 N HCl and brine The organic extracts were dried over Na₂SO₄, filtered, and concentrated. The residue was purified by flash chromatography (silica gel, 0% to 100% EtOAc in hexanes) to give compound 3 (566 mg, 98%) as a white solide m/z 576,4 (M+1).
**Compound TX63475:** To a solution of compound **3** (197 mg, 0.342 mmol) in toluene (12 mL) was added *p*-TsOH (33 mg, 0.174 mmol). The reaction was heated at 135 °C with a Dean-Stark condenser attached for 2.5h. After cooling to room temperature, the reaction mixture was extracted with EtOAc and washed with saturated NaHCO₃ and brine. The organic extracts were dried over Na₂SO₄, filtered, and concentrated. The residue was purified by flash chromatography (silica gel, 100% EtOAc in hexanes) to give compound **TX63475** (159 mg, 83%) as a white solid: ¹H NMR (400 MHz, CDCl₃) δ 8.01 (s, 1H), 5.95 (s, 1H), 3.14 (td, 1H, *J* = 4.3, 13.4 Hz), 2.94 (d, 1H, *J* = 4.7 Hz), 2.81 (t, 2H, *J* = 7.6 Hz), 2.19 (m, 1H), 1.93 (m, 3H), 1.50 (m, 13H), 1.45 (s, 3H), 1.25 (s, 3H), 1.16 (s, 3H), 1.15 (s, 3H), 1.05 (s, 3H), 1.04 (s, 3H), 0.99 (t, 3H, *J* = 7.4 Hz), 0.95 (s, 3H); m/z 558.4 (M+1).
**Compound 4:** To a solution of isobutyric acid hydrazide (153 mg, 1.50 mmol) and Et₃N (0.58 mL, 4.16 mmol) in CH₂Cl₂ (5 mL) was added a solution of compound **1** (510 mg, 1.00 mmol) in CH₃Cl₂ (5.0 mL). The reaction mixture was stirred at room temperature for 3 h. The reaction mixture was then extracted with EtOAc and washed with 1 N HCl and brine. The organic extracts were dried over Na₂SO₄, filtered, and concentrated. The residue was purified by flash chromatography (silica gel, 0% to 100% EtOAc in hexanes) to give compound 4 (525 mg, 91%) as a white solid: m/z 576.4 (M+H).
**Compound TX63476**: To a solution of compound **4** (282 mg, 0.490 mmol) in toluene (12 mL) was added *p-*TsOH (48 mg, 0.253 mmol). The reaction was heated at 135 °C with a Dean-Stark condenser attached for 1 h. After cooling to room temperature, the reaction mixture was extracted with EtOAc and washed with saturated NaHCO₃ and brine. The organic extracts were dried over Na₂SO₄, filtered, and concentrated. The residue was purified by flash chromatography (silica gel, 0% to 100% EtOAc in hexanes) to give compound **TX63476** (233 mg, 85%) as a white solid: ¹H NMR (400 MHz, CDCl₃) δ 8.02 (s, 1H), 5.95 (s, 1H), 3.17 (m, 2H), 2.99 (d, 1H, *J* = 4.7 Hz), 2.18 (dt, 1H, *J* = 4.2, 14.8 Hz), 1.90 (m, 3H), 1.45 (m, 11H), 1.45 (s, 3H), 1.37 (d, 6H, *J* = 7.0 Hz), 1.25 (s, 3H), 1.16 (s, 3H), 1.15 (s, 3H), 1.05 (s, 3H), 1.04 (s, 3H), 0.95 (s, 3H); m/z 558,3 (M+1),
**Compound 5:** To a solution of cyclopropane carboxylic acid hydrazide (155 mg, 1.55 mmol) and Et₃N (0,58 mL, 4,16 mmol) in CH₂Cl₂ (5 mL) was added a solution of compound 1 (510 mg, 1.00 mmol) in CH₂Cl₂ (5.0 mL). The reaction mixture was stirred at room temperature for 3.5 h. The reaction mixture was then extracted with EtOAc and washed with 1 N HCl and brine. The organic extracts were dried over Na₂SO₄, filtered, and concentrated. The residue was purified by flash chromatography (silica gel, 0% to 100% EtOAc in bexanes) to give compound **5** (495 mg, 86%) as a white solid: m/z 574.3 (M+1).
**Compound TX63477:** To a solution of compound **5** (288 mg, 0.502 mmol) in toluene (12 mL) was added *p*-T₃OH (55 mg, 0.289 mmol). The reaction was heated at 150 °C with a Dean-Stark condenser attached for 2.5 h. After cooling to room temperature, the reaction mixture was extracted with EtOAc and washed with saturated NaHCO₃ and brine. The organic extracts were dried over Na₂SO₄, filtered, and concentrated. The residue was purified by flash chromatography (silica gel, 0% to 100% EtOAc in hexanes) to give compound **TX63477** (231 mg, 83%) as a white solid: ¹H NMR (400 MHz, CDCl₃) δ 8.02 (s, 1H), 5.95 (s, 1H), 3.10 (td, 1H, *J* = 3.6, 13.2 Hz), 2.98 (d, 1H, *J* = 4.7 Hz), 2.12 (m, 2H), 1.90 (m, 3H), 1.4S (s, 3H), 1.43 (s, 15H), 1.25 (s, 3H), 1.18 (s, 3H), 1.16 (s, 3H), 1.04 (s, 3H), 1.04 (s, 3H), 0.94 (s, 3H), m/z 556.3 (M+1).
**Compound 6:** To a solution of methoxyacetic acid hydrazide (166 mg, 1.59 mmol) and Et₃N (0.56 mL, 4,02 mmol) in CH₂Cl₂ (5 mL) was added a solution of compound 1 (510 mg, 1.00 mmol) in CH₂Cl₂ (5.0 mL). The reaction mixture was stirred at room temperature for 4 h. The reaction mixture was then extracted with EtOAc and washed with 1 N HCl and brine. The organic extracts were dried over Na₂SO₄, filtered, and concentrated. The residue was purified by flash chromatography (silica gel, 0% to 100% EtOAc in hexanes) to give compound 6 (495 mg, 86%) as a white foam solid: m/z 578.4 (M+1).
**Compound TX63478:** To a solution of compound 6 (292 mg, 0.505 mmol) in toluene (12 mL) was added *p-*TsOH (48 mg, 0.253 mmol). The reaction was heated at 150 °C with a Dean-Stark condenser attached for 1 h. After cooling to room temperature, the reaction mixture was extracted with EtOAc and washed with saturated NaHCO₃ and brine. The organic extracts were dried over Na₂SO₄, filtered, and concentrated. The residue was purified by flash chromatography (silica gel, 0% to 100% EtOAc in hexanes) to give compound **TX63478** (158 mg, 56%) as a white solid: ¹H NMR (400 MHz, CDCl₃) δ 8.02 (s, 1H), 5.96 (s, 1H), 4.63 (s, 2H), 3.43 (s, 3H), 3,18 (1d, 1H, *J* = 4.2, 13.7 Hz), 3.01 (d, 1H, *J* = 4.7 Hz), 2.21 (m, 1H), 1,91 (m, 3H), 1.50 (m, 11H), 1,45 (s, 3H), 1.24 (s, 3H), 1.16 (s, 3H), 1.15 (s, 3H), 1.05 (s, 3H), 1.05 (s, 3H), 0.95 (s, 3H); m/z 560.3 (M+1).
**Compound** 7: To a solution of formic acid hydrazide (92 mg, 1.53 mmol) and Et₃N (0.56 mL, 4.02 mmol) in CH₂Cl₂ (5 mL) was added a solution of compound **1** (510 mg, 1.00 mmol) in CH₂Cl₂ (5.0 mL). The reaction mixture was stirred at room temperature for 1.5 h. The reaction mixture was then extracted with EtOAc and washed with 1 N HCl and brine. The organic extracts were dried over Na₂SO₄, filtered, and concentrated. The residue was purified by flash chromatography (silica gel, 0% to 100% EtOAc in hexanes) to give compound 7 (257 mg, 48%) as a white solid: m/z 534.3 (M+1).
**Compound TX63479**. To a solution of compound 7 (256 mg, 0.480 mmol) in toluene (12 mL) was added *p*-TsOH (48 mg, 0.253 mmol). The reaction was heated at 150 °C with a Dean-Stark condenser attached for 1 h. After cooling to room temperature, the reaction mixture was extracted with EtOAc and washed with saturated NaHCO₃ and brine. The organic extracts were dried over Na₂SO₄, filtered, and concentrated. The residue was purified by flash chromatography (silica gel, 0% to 100% EtOAc m hexanes) to give compound **TX63479** (120 mg, 49%) as a white solid: ¹H NMR (400 MHz, CDCl₃) δ 8.36 (s, 1H), 8.01 (s, 1H), 5.96 (s, 1H), 3.20 (td, 1H, *J* = 3.8, 13.3 Hz), 2.91 (d, 1H, *J* = 4.8 Hz), 2.23 (m, 1H), 1.93 (m, 3H), 1.46 (m, 11H), 1.44 (s, 3H), 1.25 (s, 3H), 1,15 (s, 3H), 1.14 (s, 3H), 1.06 (s, 3H), 1.05 (s, 3H), 0.96 (s, 3H), m/z S16.3 (M+1).
**Compound 8:** To a solution of acetamide oxime (113 mg, 1.53 mmol) and Et₃N (0.56 mL, 4.02 mmol) in CH₂Cl₂ (5 mL) was added a solution of compound **1** (510 mg, 1.00 mmol) in CH₂Cl₂ (5.0 mL). The reaction mixture was stirred at room temperature for 5 h. The reaction mixture was then concentrated. The residue was purified by flash chromatography (silica gel, 0% to 100% EtOAc in hexanes) to give compound **8** (510 mg, 93%) as a white solid: m/z 548.3 (M+1).
**Compound TX63501:** Compound **8** (27 mg, 0.049 mmol) was dissolved in toluene (1 mL), and the solution was heated via microwave heating at 170 °C for 10 min, followed by 200 °C for 20 min. After cooling to room temperature, the reaction mixture was concentrated. The residue was purified by flash chromatography (silica gel, 0% to 80% EtOAc in hexanes) to give compound **TX63501** (12 mg, 46%) as a white solid: ¹H NMR (400 MHz, CDCl₃) δ 8.01 (s, 1H), 5,95 (s, 1H), 3.14 (m, 1H), 3.02 (d, 1H, *J* = 4.7 Hz), 2.21 (s, 3H), 2.14 (m, 1H), 1.93 (m, 3H), 1.50 (m, 13H), 1.45 (s, 3H), 1.25 (s, 3H), 1.18 (m, 1H), 1,16 (s, 3H), 1.15 (s, 3H), 1.04 (s, 3H), 0.98 (s, 3H); m/z (M+1).
**Compound 9:** To a solution of compound **TX63199** (52 mg, 0.103 mmol) in CH₂Cl₂ (2 mL) were added acetic hydrazide (18.6 mg, 0.251 mmol), Et₃N (28 µL, 0.201 mmol), and DMAP (24.4 mg, 0.200 mmol), EDCI (40 mg, 0.209 mmol) was then added, and the reaction was stirred at room temperature for 17 h. The reaction mixture was extracted with EtOAc and washed with saturated N HCl and brine. The organic extracts were dried over Na₂SO₄, filtered, and concentrated. The residue was purified by flash chromatography (silica gel, 0% to 10% MeOH in CH₂Cl₂) to give compound **9** (33 mg, 57%) as a white solid: m/z 512.3 (M+1).
**Compound TX63593**: To a solution of compound 9 (25 mg, 0045 mmol) in toluene (1.5 mL) was added *p*-TsOH (4.8 mg, 0.025 mmol). The reaction mixture was heated via microwave heating at 125 °C for 1 h. After cooling to room temperature, the reaction mixture was extracted with BtOAc and washed with saturated NaHCO₃ and brine. The organic extracts were dried over Na₂SO₄, filtered, and concentrated. The residue was purified by flash chromatography (silica gel 20% to 100% EtOAc in hexanes) to give compound **TX63593** (11 mg, 46%) as an off-white solid: ¹H NMR (400 MHz, CDCl₃) 6 8.04 (s, 1H), 6.01 (s, 1H), 3,12 (d, 1H, *J* = 5.0 Hz), 3,12 (d, *J* = 14.1 Hx), 2.69 (d, 1H, *J* = 14.5 Hz), 2.52 (s, 3H), 2.27 (m, 1H), 1.98 (m, 2H), 1.78 (m, 3H), 1.56 (m, 3H), 1.56 (s, 3H), 1,52 (s, 3H), 1.27 (s, 3H), 1.19 (m, 7 H), 1.19 (s, 3H), 1.04 (s, 3H), 0.95 (s, 3H), 0.88 (s, 3H); m/z 544.3 (M+1).

### REFERENCES

U.S. Patent No. 7,915,402
U.S. Patent No. 7,943,778
U.S. Patent No. 8,071,632
U.S. Patent No. 8,124,799
U.S. Patent No 8,129,29
U.S. Patent No. 8,338,618
Abraham and Kappas, Free Radical Biol. Med, 39:1-25, 2005,
Ahmad et al., Cancer Res., 68:2920-2926, 2008.
Ahmad et al., J. Biol. Chem., 281:35764-9, 2006.
Araujo et al., J. Immunol., 171(3):1572-1580, 2003.
Bach, Hum. Immunol., 67(6):430-432, 2006.
Chauhan and Chauhan, Pathophysiology, 13(3): 171-181 2006.
Dickerson et al., Prog Neuropsychopharmacol Biol. Psychiatry, March 6, 2007.
Dinkova-Kostova et al., Proc. Natl. Acad. Sci. USA, 102(12):4584-4589, 2005.
Dudhgaonkar et al., Eur. J. Pain, 10(7):573-9, 2006.
Forstermann, Biol. Chem., 387:1521, 2006,
Handbook of Pharmaceutical Salts: Properties, and Use, Stahl and Wermuth Eds.), Verlag Helvetica Chimica Acta, 2002.
Hanson et al., BMC Medical Genetics, 6(7), 2005.
Honda et al. Bioorg Med. Chem. Lett., 12:1027-1030, 2002.
Honda et al., J. Med. Chem., 43:4233-4246, 2000a.
Honda, et al., J. Med. Chem., 43:1866-1877, 2000b.
Honda et al., Bioorg. Med. Chem. lett., 7:1623-1628, 1997.
Honda et al., Bioorg. Med. Chem. Lett., 9(24):3429-3434, 1999.
Honda et al., Bioorg. Med. Chem. Lett., 8(19):2711-2714, 1998.
Honda et al, Bioorg. Med. Chem. Lett., 16(24):6306-6309, 2006.
Hong et al., Clin Cancer Res, 18(12):3396-406, 2012.
Ishikawa et al., Circulation, 104(15):1831-1836, 2001.
Kawakami et al., Brain Dev., 28(4):243-246, 2006.
Kendall-Tackett, Trauma Violence Abuse, 8(2):117-126, 2007.
Kruger et al., J Pharmacol Exp. Ther., 319(3):1144-1152, 2006.
Lee et al., Glia., 55(7):712-22, 2007.
Lenex et al., Mol. Psychiatry, 12(6):572-80, 2007.
Liby et al., Cancer Res., 65(11):4789-4798, 2005.
Liby et al., Nat. Rev. Cancer, 7(5):357-356, 2007a.
Liby et al., Mol. Cancer Ther., 6(7):2113-9, 2007b.
Liby *et al*., 2007b
Liu et al., FASEB J., 20(2):207-216, 2006.
Lu et al., J. Clin. Invest., 121(10):4015-29, 2011.
March's Advanced Organic Chemistry: Reactions, Mechanisms, and Structure, 2007.
Mclever et al., Pain, 120(1-2):161-9, 2005.
Morris et al., J. Mol. Med., 80(2):96-104, 2002.
Morse and Choi, Am. J. Respir. Crit. Care Med., 72(6):660-670, 2005.
Morse and Choi, Am. J. Respir, Crit, Care Med., 27(1):8-16, 2002.
Pall, Med. Hypoth., 69:821-825, 2007.
Pergola et al., N Engl J Med, 365:327-336, 2011.
Place et al., Clin. Cancer Res., 9(7):2798-806, 2003.
Rajakariar et al., Proc. Nati. Acad. Sci. USA, 104(52):20979-84, 2007.
Ross et al., Am. J. Clin. Pathol., 120(Suppl):S53-71, 2003.
Ross et al., Expert Rev. Mol. Diagn., 3(5):573-585, 2003.
Ruster et al., Scand. J. Rheumatol., 34(6):460-3, 2005.
Sacerdoti et al., Curr Neurovase Res. 2(2):103-111, 2005.
Salvemini et al., J. Clin. Invest., 93(5):1940-1947, 1994.
Sarchielli et al., Cephalalgia, 26(9):1071-1079, 2006.
Satoh et al., Proc. Natl. Acad. Sci. USA, 103(3):768-773, 2006,
Schulz et al., Antioxid. Redox, Sig., 10:115, 2008.
Strejan et al, J. Neuroimmunol., 7:27, 1984,
Suh et al., Cancer Res., 58:717-723, 1998.
Suh et al., Cancer Res., 59(2):336-341, 1999.
Szabo et al., Nature Rev. Drug Disc., 6:662-680, 2007.
Takahashi et al., Cancer Res., 57:1233-1237, 1997,
Tamir and Tannebaum, Biochim. Biophys. Acta, 1288:P31-F36, 1996.
Xie et al., J. Biol. Chem., 270(12):6894-6900, 1995.
Zhou et al., Am. J. Pathol., 166(1):27-37, 2005.

## Claims

1. A compound of the formula: 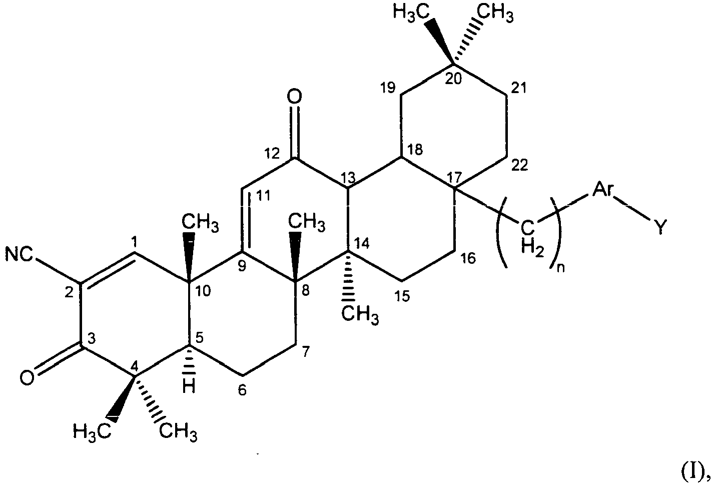 wherein:
n is 0-3;
Ar is heteroarenediyl_{(C≤8)} or a substituted version thereof; and
Y is:
hydrogen, hydroxy, halo, amino, or cyano or -NCO; or
alkyl_{(C≤8)}, cycloalkyl_{(C≤8)}, alkenyl_{(C≤8)}, alkynyl_{(C≤8)}, aryl_{(C≤12)}, aralkyl_{(C≤12)}, heteroaryl_{(C≤8)}, heterocycloalkyl_{(C≤12)}, acyl_{(C≤12)}, alkoxy_{(C≤8)}, aryloxy_{(C≤12)}, acyloxy_{(C≤8)}, alkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, or substituted versions of any of these groups;
or a pharmaceutically acceptable salt thereof;
and wherein
in the substituted versions of alkyl_{(C≤8)}, cycloalkyl_{(C≤8)}, alkenyl_{(C≤8)}, alkynyl_{(C≤8)}, aryl_{(C≤8)}, aralkyl_{(C≤12)}, heteroaryl_{(C≤8)}, acyl_{(C≤12)}, alkoxy_{(C≤8)}, aryloxy_{(C≤12)}, acyloxy_{(C≤8)}, alkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)} one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, or -S(O)₂NH₂
in the substituted version of heterocycloalkyl_{(C≤8)}, one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, -S(O)₂NH₂ or -C(O)OC(CH₃)_{3;}
the term "aryl" refers to a monovalent aromatic group with an aromatic carbon atom as the point of attachment which forms part of a one or more six-membered aromatic ring structure, wherein the ring atoms are all carbon, and wherein, if more than one ring is present, the rings may be fused or unfused, and wherein one or more alkyl or aralkyl groups may be attached to the first aromatic ring or any additional aromatic ring present, if permitted by the carbon number limitation;
the term "heteroaryl" refers to a monovalent aromatic group with an aromatic carbon atom or nitrogen atom as the point of attachment, said carbon atom or nitrogen atom forming part of one or more aromatic ring structures wherein at least one of the ring atoms is nitrogen, oxygen or sulfur, wherein the heteroaryl group consists of no atoms other than carbon, hydrogen, aromatic nitrogen, aromatic oxygen and aromatic sulfur, wherein if more than one ring is present, the rings may be fused or unfused, and wherein one or more alkyl, aryl, and/or aralkyl groups may be attached to the aromatic ring or aromatic ring system, if permitted by the carbon number limitation;
the term "heteroarenediyl" refers to a divalent aromatic group, with two aromatic carbon atoms, two aromatic nitrogen atoms, or one aromatic carbon atom and one aromatic nitrogen atom as the two points of attachment, said atoms forming part of one or more aromatic ring structure(s) wherein at least one of the ring atoms is nitrogen, oxygen or sulfur, wherein the divalent group consists of no atoms other than carbon, hydrogen, aromatic nitrogen, aromatic oxygen and aromatic sulfur, wherein, if more than one ring is present, the rings may be fused or unfused, wherein unfused rings may be connected via one or more of the following: a covalent bond, alkenediyl, oar alkellediyl groups, if permitted by the carbon number limitation, and wherein one or more alkyl, aryl, and/or aralkyl groups may be attached to the aromatic ring or aromatic ring system, if permitted by the carbon number limitation;
the term "heterocycloalkyl" refers to a monovalent non-aromatic group with a carbon atom or nitrogen atom as the point of attachment, said carbon atom or nitrogen atom forming part of one or more non-aromatic ring structures wherein at least one of the ring atoms is nitrogen, oxygen or sulfur, wherein the heterocycloalkyl group consists of no atoms other than carbon, hydrogen, nitrogen, oxygen and sulfur, wherein, if more than one ring is present, the rings may be fused or unfused, wherein, the one or more alkyl groups may be attached to the ring or ring system, if permitted by the carbon number limitation, and wherein one or more double bonds may be present in the ring or ring system, provided that the resulting group remains non-aromatic;
the term "acyl" refers to the group -C(O)R, in which R is a hydrogen, alkyl, aryl, aralkyl or heteroaryl; and
the term "dialkylamino" refers to the group -NRR', in which R and R' can be the same or different alkyl groups, or R and R' can be taken together to represent an alkanediyl.

2. The compound of claim 1, wherein Y is -H.

3. The compound of claim 1, wherein Y is aikyt_{(C≤4)} or cycloalkyl_{(C≤4)}.

4. The compound of claim 3, wherein Y is methyl, *n*-propyl, isopropyl or cyclopropyl.

5. The compound of claim 1, wherein Y is substituted alkyl_{(C≤4)} or substituted cycloalkyl_{(C≤4)}.

6. The compound of claim 5, wherein Y is methoxymethyl.

7. The compound according to any one of claims 1-6, wherein Ar is 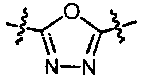 or

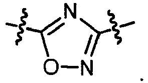

8. The compound according to any one of claims 1-7, wherein n = 0.

9. The compound according to any one of claims 1-7, wherein n = 1.

10. The compound of claim 1, further defined as: 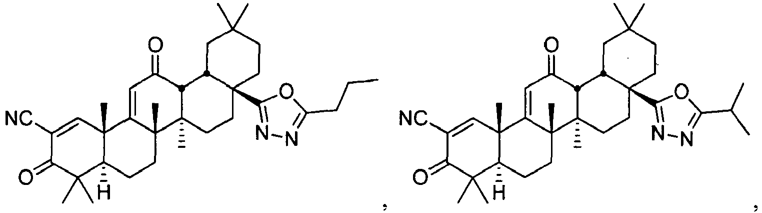 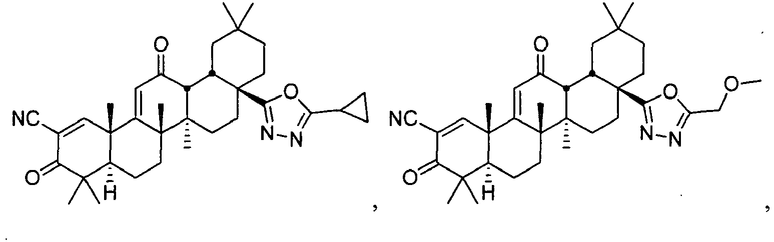 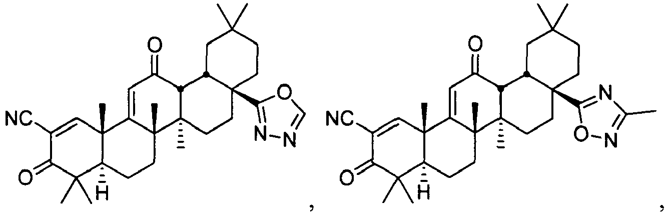 or 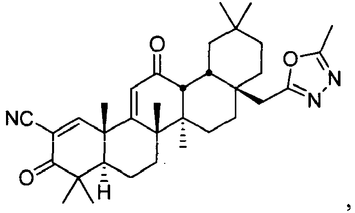 or a pharmaceutically acceptable salt thereof.

11. The compound of claim 1, further defined as: 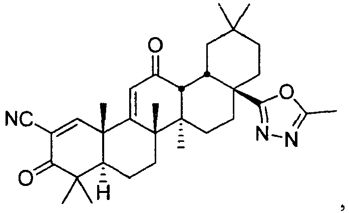 or a pharmaceutically acceptable salt thereof.

12. The compound of claim 1, further defined as: 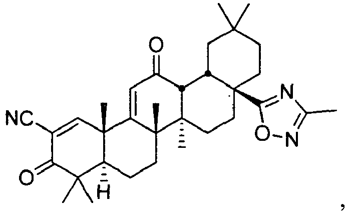 or a pharmaceutically acceptable salt thereof.

13. A pharmaceutical composition comprising:
a) a compound according to any one of claims 1-12; and
b) an excipient.

14. A compound according to any one of claims 1-12 for use as a medicament.

15. The compound for the use of claim 14, wherein the medicament is for treating a patient for inflammation and/or oxidative stress.

## Patentansprüche

1. Eine Verbindung der Formel: 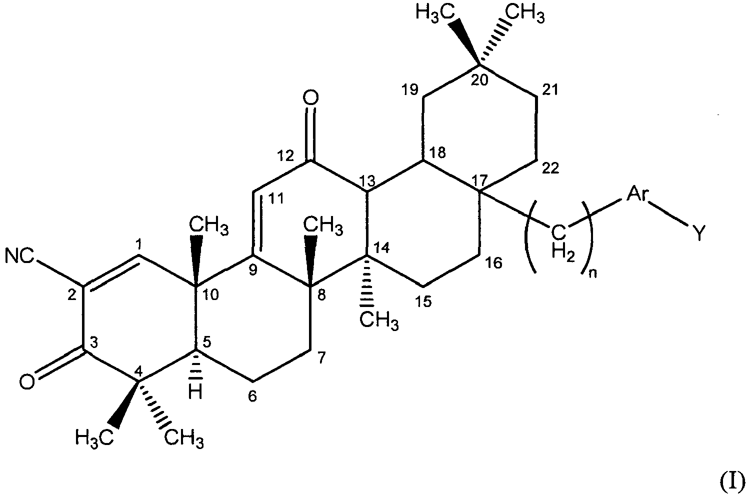 wobei:
n gleich 0-3 ist;
Ar für Heteroarendiyl_{(C≤8)} oder eine substituierte Version davon steht; und
Y:
Wasserstoff, Hydroxy, Halogen, Amino oder Cyano oder NCO; oder
Alkyl_{(C≤8)}, Cycloalkyl_{(C≤8)}, Alkenyl_{(C≤8)}, Alkinyl_{(C≤8)}, Aryl_{(C≤12)}, Aralkyl_{(C≤12)}, Heteroaryl_{(C≤8)}, Heterocycloalkyl_{(C≤12)}, Acyl_{(C≤12)}, Alkoxy_{(C≤8)}, Aryloxy_{(C≤12)}, Acyloxy_{(C≤8)}, Alkylamino_{(C≤8)}, Dialkylamino_{(C≤8)}, Arylamino_{(C≤8)}, Aralkylamino_{(C≤8)}, Alkylthio_{(C≤8)}, Acylthio_{(C≤8)}, Alkylsulfonylamino_{(C≤8)} oder substituierte Versionen einer dieser Gruppen ist;
oder ein pharmazeutisch verträgliches Salz davon;
und wobei
in den substituierten Versionen von Alkyl_{(C≤8)}, Cycloalkyl_{(C≤8)}, Alkenyl_{(C≤8)}, Alkinyl_{(C≤8)}, Aryl_{(C≤12)}, Aralkyl_{(C≤12)}, Heteroaryl_{(C≤8)}, Acyl_{(C≤12)}, Alkoxy_{(C≤8)}, Aryloxy_{(C≤12)}, Acyloxy_{(C≤8)}, Alkylamino_{(C≤8)}, Dialkylamino_{(C≤8)}, Arylamino_{(C≤8)}, Aralkylamino_{(C≤8)}, Alkylthio_{(C≤8)}, Acylthio_{(C≤8)}, Alkylsulfonylamino_{(C≤8)} ein oder mehrere Wasserstoffatome unabhängig durch -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃ oder -S(O)₂NH₂ ersetzt wurden,
in der substituierten Version von Heterocycloalkyl_{(C≤12)} ein oder mehrere Wasserstoffatome unabhängig durch -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, -S(O)₂NH₂ oder -C(O)OC(CH₃)₃ ersetzt wurden;
der Begriff "Aryl" sich auf eine monovalente aromatische Gruppe mit einem aromatischen Kohlenstoffatom als Verknüpfungspunkt bezieht, welcher Teil einer einfach oder mehrfach sechsgliedrigen Ringstruktur ist, wobei die Ringatome alle Kohlenstoff sind, und wobei, falls mehr als ein Ring vorhanden ist, die Ringe kondensiert oder nicht kondensiert sein können und wobei eine oder mehrere Alkyl- oder Aralkylgruppen an den ersten aromatischen Ring oder jeden beliebigen zusätzlich vorhandenen aromatischen Ring gebunden sein können, falls die Kohlenstoffzahlbegrenzung dies zulässt;
der Begriff "Heteroaryl" sich auf eine monovalente aromatische Gruppe mit einem aromatischen Kohlenstoffatom oder Stickstoffatom als Verknüpfungspunkt bezieht, wobei das Kohlenstoffatom oder das Stickstoffatom Teil einer oder mehrerer aromatischer Ringstrukturen ist, wobei mindestens eines der Ringatome Stickstoff, Sauerstoff oder Schwefel ist, wobei die Heteroarylgruppe aus keinen weiteren Atomen neben Kohlenstoff-, Wasserstoff-, aromatischen Stickstoff-, aromatischen Sauerstoff-und aromatischen Schwefelatomen besteht, wobei, falls mehr als ein Ring vorhanden ist, die Ringe kondensiert oder unkondensiert sein können, und wobei eine oder mehrere Alkyl-, Aryl-, und/oder Aralkylgruppen an den aromatischen Ring oder das aromatische Ringsystem gebunden sein können, falls die Kohlenstoffzahlbegrenzung dies zulässt;
der Begriff "Heteroarendiyl" sich auf eine divalente aromatische Gruppe mit zwei aromatischen Kohlenstoffatomen, zwei aromatischen Stickstoffatomen oder einem aromatischen Kohlenstoffatom und einem aromatischen Stickstoffatom als die zwei Verknüpfungspunkte bezieht, wobei die Atome Teil einer oder mehrerer aromatischer Ringstruktur(en) sind, wobei mindestens eines der Ringatome Stickstoff, Sauerstoff oder Schwefel ist, wobei die divalente Gruppe aus keinen weiteren Atomen neben Kohlenstoff-, Wasserstoff-, aromatischen Stickstoff-, aromatischen Sauerstoff- und aromatischen Schwefelatomen besteht, wobei, falls mehr als ein Ring vorhanden ist, die Ringe kondensiert oder unkondensiert sein können, wobei unkondensierte Ringe über eines oder mehrere der folgenden verbunden sein können: eine kovalente Bindung, Alkandiyl oder Alkendiylgruppen, falls die Kohlenstoffzahlbegrenzung dies zulässt, und wobei eine oder mehrere Alkyl-, Aryl-, und/oder Aralkylgruppen an den aromatischen Ring oder das aromatische Ringsystem gebunden sein können, falls die Kohlenstoffzahlbegrenzung dies zulässt;
der Begriff "Heterocycloalkyl" sich auf eine monovalente nicht-aromatische Gruppe mit einem Kohlenstoffatom oder einem Stickstoffatom als Verknüpfungspunkt bezieht, wobei das Kohlenstoffatom oder das Stickstoffatom Teil einer oder mehrerer nichtaromatischer Ringstrukturen ist, wobei mindestens eines der Ringatome Stickstoff, Sauerstoff oder Schwefel ist, wobei die Heterocycloalkylgruppe aus keinen weiteren Atomen neben Kohlenstoff-, Wasserstoff-, Stickstoff-, Sauerstoff- und Schwefelatomen besteht, wobei, falls mehr als ein Ring vorhanden ist, die Ringe kondensiert oder unkondensiert sein können, wobei die eine oder mehreren Alkylgruppen an den Ring oder das Ringsystem gebunden sein können, falls die Kohlenstoffzahlbegrenzung dies zulässt, und wobei eine oder mehrere Doppelbindungen in dem Ring oder dem Ringsystem vorhanden sein können, vorausgesetzt, dass die daraus entstandene Gruppe nicht-aromatisch bleibt;
der Begriff "Acyl" sich auf die Gruppe -C(O)R bezieht, in welcher R Wasserstoff, Alkyl, Aryl, Aralkyl oder Heteroaryl darstellt; und
der Begriff "Dialkylamino" sich auf die Gruppe -NRR' bezieht, in welcher R und R' die gleichen oder verschiedene Alkylgruppen sein können, oder R und R' zusammen genommen werden können, um ein Alkandiyl darzustellen.

2. Die Verbindung gemäß Anspruch 1, wobei Y gleich -H ist.

3. Die Verbindung gemäß Anspruch 1, wobei Y Alkyl_{(C≤4)} oder Cycloalkyl_{(C≤4)} darstellt.

4. Die Verbindung gemäß Anspruch 3, wobei Y Methyl, *n*-Propyl, Isopropyl oder Cyclopropyl darstellt.

5. Die Verbindung gemäß Anspruch 1, wobei Y substituiertes Alkyl_{(C≤4)} oder substituiertes Cycloalkyl_{(C≤4)} darstellt.

6. Die Verbindung gemäß Anspruch 5, wobei Y Methoxymethyl darstellt.

7. Die Verbindung gemäß einem der Ansprüche 1-6, wobei Ar gleich 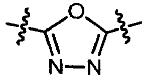 oder 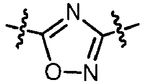 ist.

8. Die Verbindung gemäß einem der Ansprüche 1-7, wobei n = 0.

9. Die Verbindung gemäß einem der Ansprüche 1-7, wobei n = 1.

10. Die Verbindung gemäß Anspruch 1, ferner definiert als: 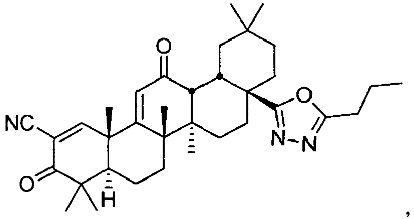 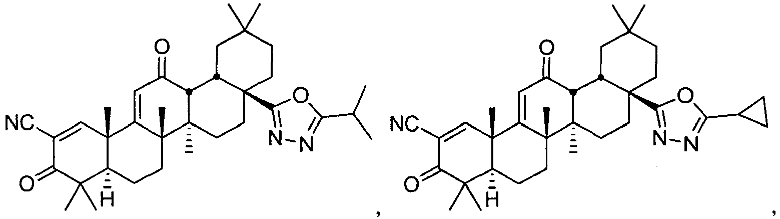 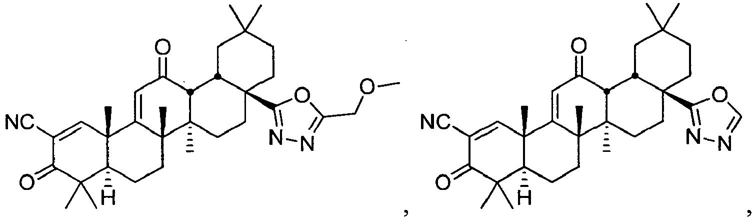 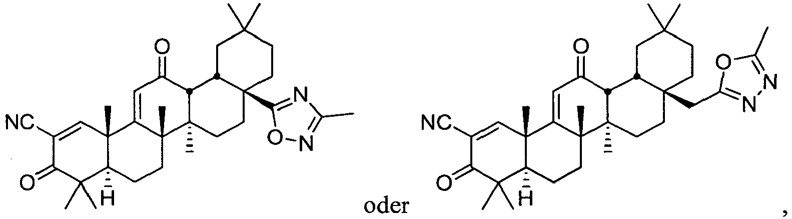 oder ein pharmazeutisch verträgliches Salz davon.

11. Die Verbindung gemäß Anspruch 1, ferner definiert als: 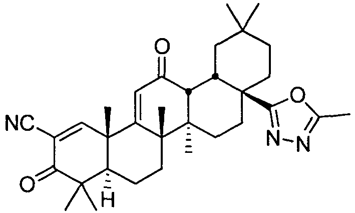 oder ein pharmazeutisch verträgliches Salz davon.

12. Die Verbindung gemäß Anspruch 1, ferner definiert als: 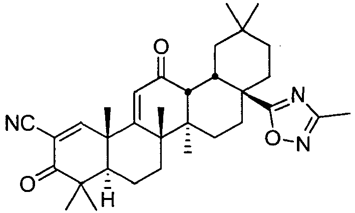 oder ein pharmazeutisch verträgliches Salz davon.

13. Ein Arzneimittel, umfassend:
a) eine Verbindung gemäß einem der Ansprüche 1-12; und
b) einen Exzipienten.

14. Eine Verbindung gemäß einem der Ansprüche 1-12 zur Verwendung als Medikament.

15. Die Verbindung zur Verwendung gemäß Anspruch 14, wobei das Medikament zur Behandlung eines Patienten mit Entzündung und/oder oxidativem Stress dient.

## Revendications

1. Composé de formule : 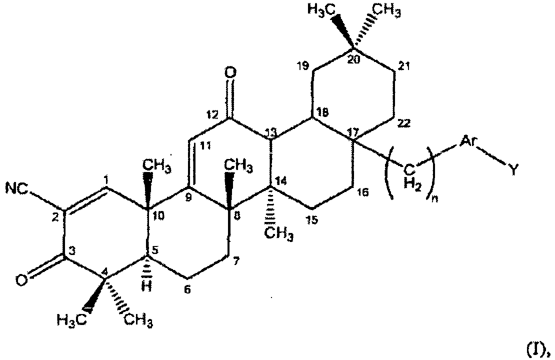 dans laquelle
n est de 0 à 3 ;
Ar est un hétéroarènediyle_{(C≤8)}, ou une forme substituée de celui-ci ; et Y est :
un atome d'hydrogène, un hydroxy, halogéno, amino, ou cyano ou -NCO ; ou un alkyle_{(C≤8)}, cycloalkyle_{(C≤8)}, alcényle_{(C≤8)}, alcynyle_{(C≤8)}, aryle_{(C≤12)}, aralkyle_{(C≤12)}, hétéroaryle_{(C≤8)}, hétérocycloalkyle_{(C≤12)}, acyle_{(C≤12)}, alcoxy_{(C≤8)}, aryloxy_{(C≤12)}, acyloxy_{(C≤8)}, alkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, ou des formes substituées de l'un quelconque de ces groupes ;
ou un sel pharmaceutiquement acceptable de celui-ci ; et où
dans les formes substituées des groupes alkyle_{(C≤8)}, cycloalkyle_{(C≤8)}, alcényle_{(C≤8)}, alcynyle_{(C≤8)}, aryle_{(C≤12)}, aralkyle_{(C≤12)}, hétéroaryle_{(C≤8)}, acyle_{(C≤12)}, alcoxy_{(C≤8)}, aryloxy_{(C≤12)}, acyloxy_{(C≤8)}, alkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, un ou plusieurs atomes d'hydrogène ont été indépendamment remplacés par -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, ou -S(O)₂NH₂
dans la forme substituée de l'hétérocycloalkyle_{(C≤12)}, un ou plusieurs atomes d'hydrogène ont été indépendamment remplacés par -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -OC(O)CH₃, -S(O)₂NH₂ ou -C(O)OC(CH₃)₃,
le terme « aryle » désigne un groupe aromatique monovalent ayant un atome de carbone aromatique qui fait partie d'une ou de plusieurs structures de cycles aromatiques à six chaînons comme point d'attachement, dans lequel les atomes de cycles sont tous des carbones, et dans lequel, si plus d'un cycle est présent, les cycles peuvent être condensés ou non, et dans lequel un ou plusieurs groupes alkyle ou aralkyle peuvent être liés au premier cycle aromatique ou à tout cycle aromatique supplémentaire présent, si autorisé par la limite du nombre de carbones ;
le terme « hétéroaryle » désigne un groupe aromatique monovalent ayant un atome de carbone aromatique ou un atome d'azote comme point d'attachement, ledit atome de carbone ou atome d'azote faisant partie d'une ou de plusieurs structures de cycles aromatiques, dans lequel au moins un des atomes de cycles est un atome d'azote, d'oxygène ou de soufre, dans lequel le groupe hétéroaryle ne se compose d'aucun atome autre que carbone, hydrogène, azote aromatique, oxygène aromatique et soufre aromatique, dans lequel si plus d'un cycle est présent, les cycles peuvent être condensés ou non, et dans lequel un ou plusieurs groupes alkyle, aryle, et/ou aralkyle peuvent être liés au cycle aromatique ou au système de cycles aromatiques, si autorisé par la limite du nombre de carbones ;
le terme « hétéroarènediyle » désigne un groupe aromatique divalent, ayant deux atomes de carbone aromatiques, deux atomes d'azote aromatiques, ou un atome de carbone aromatique et un atome d'azote aromatique comme les deux points d'attachement, lesdits atomes faisant partie d'une ou de plusieurs structures de cycles aromatiques, dans lequel au moins un des atomes de cycles est un atome d'azote, d'oxygène ou de soufre, dans lequel le groupe divalent ne se compose d'aucun atome autre que carbone, hydrogène, azote aromatique, oxygène aromatique et soufre aromatique, dans lequel si plus d'un cycle est présent, les cycles peuvent être condensés ou non, dans lequel des cycles non condensés peuvent être liés par l'un ou plusieurs des moyens suivants : une liaison covalente, des groupes alcanediyle ou alcènediyle, si autorisé par la limite du nombre de carbones, et dans lequel un ou plusieurs groupes alkyle, aryle, et/ou aralkyle peuvent être liés au cycle aromatique ou au système de cycles aromatiques, si autorisé par la limite du nombre de carbones ;
le terme « hétérocycloalkyle » désigne un groupe non aromatique monovalent ayant un atome de carbone ou un atome d'azote comme point d'attachement, ledit atome de carbone ou atome d'azote faisant partie d'une ou de plusieurs structures de cycles non aromatiques, dans lequel au moins un des atomes de cycles est un atome d'azote, d'oxygène ou de soufre, dans lequel le groupe hétérocycloalkyle ne se compose d'aucun atome autre que carbone, hydrogène, azote, oxygène et soufre, dans lequel, si plus d'un cycle est présent, les cycles peuvent être condensés ou non, dans lequel le ou les groupes alkyle peuvent être liés au cycle ou au système de cycles, si autorisé par la limite du nombre de carbones, et dans lequel une ou plusieurs doubles liaisons peuvent être présentes dans le cycle ou système de cycles, à condition que le groupe obtenu reste non aromatique ;
le terme « acyle » désigne le groupe -C(O)R, où R est un atome d'hydrogène, un alkyle, aryle, aralkyle ou hétéroaryle ; et
le terme « dialkylamino » désigne le groupe -NRR', où R et R' peuvent être des groupes alkyle identiques ou différents, ou R et R' peuvent être pris ensemble pour représenter un alcanediyle.

2. Composé selon la revendication 1, dans lequel Y est -H.

3. Composé selon la revendication 1, dans lequel Y est un alkyle_{(C≤4)} ou un cycloalkyle_{(C≤4)}.

4. Composé selon la revendication 3, dans lequel Y est un méthyle, *n*-propyle, isopropyle ou cyclopropyle.

5. Composé selon la revendication 1, dans lequel Y est un alkyle_{(C≤4)} substitué ou un cycloalkyle_{(C≤4)} substitué.

6. Composé selon la revendication 5, dans lequel Y est un méthoxyméthyle.

7. Composé selon l'une quelconque des revendications 1 à 6, dans lequel Ar est

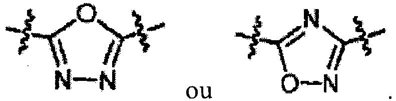

8. Composé selon l'une quelconque des revendications 1 à 7, dans lequel n = 0.

9. Composé selon l'une quelconque des revendications 1 à 7, dans lequel n = 1.

10. Composé selon la revendication 1, défini en outre par les formules : 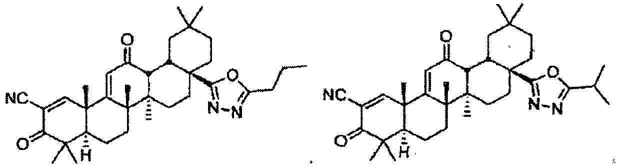 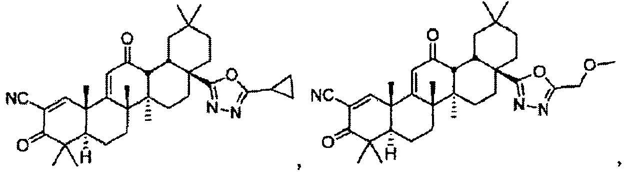 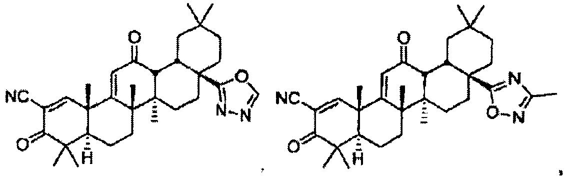 ou 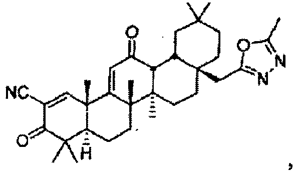 ou un sel acceptable sur le plan pharmaceutique de celui-ci.

11. Composé selon la revendication 1, défini en outre par la formule : 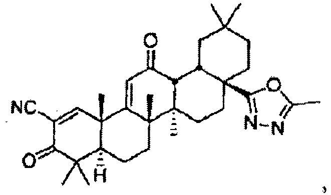 ou un sel acceptable sur le plan pharmaceutique de celui-ci.

12. Composé selon la revendication 1, défini en outre par la formule : 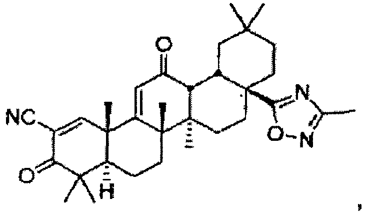 ou un sel acceptable sur le plan pharmaceutique de celui-ci.

13. Composition pharmaceutique comprenant :
a) un composé selon l'une quelconque des revendications 1 à 12 ; et
b) un excipient.

14. Composé selon l'une quelconque des revendications 1 à 12 pour une utilisation comme médicament.

15. Composé pour son utilisation selon la revendication 14, dans lequel le médicament est destiné à traiter l'inflammation et/ou le stress oxydatif chez un patient.
